# EUROPEAN PATENT APPLICATION

(11) **EP 3 789 482 A1**
(43) Date of publication of application: **10.03.2021**
(21) Application number: 19796360.6
(22) Date of filing: 30.04.2019
(51) Int. Cl.: C12N 5/071, A61K 48/00, A61P 37/02

(54) **TECHNIQUE FOR REGULATING JAK-STAT PATHWAY TO ALLOW CELL DIFFERENTIATION, DEDIFFERENTIATION, AND REJUVENATION, AND APPLICATIONS OF TECHNIQUE**

(30) Priority: 01.05.2018 CN 201810407253
(71) Applicant: Shenzhen Alpha Biopharmaceutical Co., Ltd., Shenzhen,Guangdong 518000 (CN)
(72) Inventor: HU, Min, Kunming, Yunnan 650214 (CN); LI, Yanjiao, Kunming, Yunnan 650214 (CN); HU, Junyuan, Shenzhen, Guangdong 518000 (CN)
(74) Representative: Vitina, Maruta
(86) International application number: PCT/CN2019/085186
(87) International publication number: WO 2019/210851

(57) **Abstract**

A method of regulating cell differentiation, dedifferentiation, transdifferentiation, rejuvenation, aging and apoptosis by regulating Jak-Stat signaling pathway and uses thereof. In the method, a gene or protein target involved in the JAK-STAT signaling pathway is regulated quantitatively or in a timing manner using a small molecular combination, a cytokine or recombinant protein combination, gene editing or transgenic technique to achieve the rejuvenation and/or obtain different lineages of cells. This application further provides cell products generated in the method or their derivatives for there programming and immunoregulation of cells, tissues, organs and organisms, construction of tissue engineering materials, repairing of injured, aged and degenerated mammalian tissues and organs and delaying and reversing of the aging process of cells, tissues, organs and organisms.

## Description

### TECHNICAL FIELD

The present application relates to cell biology, in particular to a technique of regulating JAK-STAT signaling pathway to allow cell differentiation, dedifferentiation, and rejuvenation, and applications of the technique, and more particularly to a method of regulating the differentiation, dedifferentiation, rejuvenation, aging and apoptosis of cells and prolonging the lifespan by regulating the JAK-STAT signaling pathway, cell products prepared thereby and uses thereof.

### BACKGROUND

Stem cells are considered as "Holy Grail" in the regenerative medicine and anti-aging fields. Generally, the aging of an organism is accompanied by the aging of stem cells, which will bring the malignant transformation or degeneration of various organs including bone, cartilage, heart, muscle, brain, skin, pancreas, liver, kidney and gastrointestinal tract. Moreover, the aging may also cause the dysfunction of immune system. In fact, the chronic inflammation is also a cause of the degeneration and aging of tissues and organs. The aged cells usually suffer DNA damage or mutations, telomere shortening, abnormal epigenetics, redox, and energy metabolism, declined proliferation capacity and increased death rate. The aged stem cells tend to lose the potential to differentiate into certain lineages while biasing towards others. For example, it is well known that the bone marrow mesenchymal stem cells from an elderly individual have decreased osteogenic or chondrogenic potential but increased adipogenic potential. Therefore, the bone marrow derived from an elderly person is often filled with fat tissues and thus called "yellow bone marrow". Similarly, the neural stem cells of the elderly will also tend to differentiate into astrocytes rather than neurons, which is believed to be related to the decline in cognitive ability of the elderly.

Stem cells, especially mesenchymal stem cells, have exhibited strong potential in the treatment or intervention of the aging process and related diseases since they are readily available, expandable and pluripotent, and can release growth factors and regulate the immune system. Currently, the mesenchymal stem cells have been widely used in the clinical treatment of various diseases, such as graft-versus-host disease (GVHD), multiple sclerosis (MS), amyotrophic lateral sclerosis (ALS), spinal cord injury (SCI), lupus erythematosus (LE), arthritis and aging. Among the mesenchymal stem cells (MSCs), umbilical cord mesenchymal stem cells are widely considered to be suitable for the allogeneic uses, but the long-term/repetitive use of "non-self" cells presents a clinical risk. By contrast, autologous stem cell transplantation is considered safer, but unfortunately, the mesenchymal stem cells also become aged as the individual ages, and the aged MSCs are greatly limited in clinical application due to the loss of many important functions. Induced pluripotent stem (iPS) cells are a class of young cells that can be obtained from the elderly, and have been deemed as a desired autologous cell source of cells for treatment. However, the preparation of iPS cells involves poor induction efficiency and the introduction of foreign genes, which is often accompanied by genetic variation, limiting the clinical promotion. In addition, genetic modification has been recently used as another tool in the cell rejuvenation, but this method still has the risk of off-target and tumorigenesis. Therefore, it is of considerable significance to targetedly develop a preparation method of self-rejuvenated, safe and regenerative cells for delaying and reversing the process of human aging and repairing the structure or function of tissues and organs.

### SUMMARY

An object of this application is to provide a technique of regulating JAK-STAT signaling pathway to allow cell differentiation, dedifferentiation, and rejuvenation, and applications of the technique, especially a method of regulating cell differentiation, dedifferentiation, transdifferentiation, rejuvenation, aging and apoptosis by regulating JAK-STAT (Janus kinase-signal transducer and activator of transcription) signaling pathway to reverse the aging process and prolong the lifespan. Moreover, the cells prepared through the above method such as regenerative fibroblast (rFib) and induced rejuvenated mesenchymal stem cell (irMSC) can be employed to prevent, delay and reverse the human aging process and repair the structure or function of tissues and organs. The regenerative fibroblasts prepared herein have both the characteristics of skin fibroblasts and mesenchymal stem cells, and thus are also named induced and rejuvenated mesenchymal stem cells (irMSCs) or induced mesenchymal stem cells (iMSCs) (uniformly referring to as rFib herein).

Technical solutions of this application are described as follows.

In a first aspect, this application provides a method of regulating the differentiation, dedifferentiation and rejuvenation of target cells, comprising:
regulating an expression of a gene or protein target involved in JAK-STAT signaling pathway to activate or inhibit the JAK-STAT signaling pathway quantitatively and/or in a timing manner in the target cells;
wherein the gene or protein target involved in the JAK-STAT signaling pathway is selected from the group consisting of: *CXCL2* (Accession No: AY577905.1), *SOS1* (Accession No: NM_005633.3), *STAT5B* (Accession No: NM_012448.3), *JAK1* (Accession No: NM_001321857.1), *JAK3* (Accession No: NM_000215.3), *SOCS3* (Accession No: NM_003955.4), *IL6ST* (Accession No: NM_001243835.1), *STAT1* (Accession No: NM_007315.3), *STAT2* (Accession No: NM_198332.1), *STAT3* (Accession No: NM_213662.1), *STAT4* (Accession No:NM_001243835.1), *STAT6* (Accession No: NM_001178081.1), *STAT5A* (Accession No: NM_001288720.1), *IRF9* (Accession No: NM_006084.4), *IL6* (Accession No: XM_005249745.5), *IL6R* (Accession No: NM_181359.2), *IL2* (Accession No: NM_000586.3) (such as *IL2A* and *IL2B*), *PRKCD* (Accession No: NM_001354679.1), *CXCL12* (Accession No: NM_000609.6), *CXCR4* (Accession No: NM_003467.2), *JAK2* (Accession No: NM_004972.3), *IL15RA* (Accession No: NM_001351095.1), *IL20RB* (Accession No: XM_006713665.4), *GHR* (Accession No: NM_001242406.2), *PRLR* (Accession No: NM_001204314.2) and a combination thereof.

In an embodiment, the target cells are derived from mammals, such as humans, mice, monkeys and pigs, and are selected from the group consisting of fibroblasts, epithelial cells, adipocytes, blood cells, mesenchymal stem cells, nerve cells, muscle cells, cardiomyocytes, smooth muscle cells, vascular endothelial cells, induced pluripotent stem cells, embryonic stem cells, osteoblasts, chondrocytes and osteoclasts. The rejuvenated mesenchymal stem cells prepared herein are derived from the target cells and other cell types can also be produced in the process associated with the regulation of the JAK-STAT signaling pathway, where the process associated with the regulation of the JAK-STAT signaling pathway includes differentiation, dedifferentiation, transdifferentiation, rejuvenation, aging and apoptosis.

In an embodiment, the quantitative activation or inhibition of the JAK-STAT signaling pathway indicates that the expression of at least one of the gene or protein targets involved in the JAK-STAT signaling pathway in the rejuvenated and regenerative fibroblasts or irMSC is up-regulated or down-regulated by 1-300 times relative to the target cells.

In an embodiment, the activation or inhibition of the JAK-STAT signaling pathway in a timing manner indicates that at least one of the gene or protein targets involved in the JAK-STAT signaling pathway in the target cells is regulated to experience high expression, low expression or no expression for 24 hours-220 days, and the resulting cells can maintain the high expression, low expression or no expression of the at least one of the gene or protein targets in the long term, or recover to be the same as the target cells in the expression level.

In an embodiment, the activation or inhibition of the JAK-STAT signaling pathway is performed by regulating at least one of the following pathways or targets: NOD-like receptor signaling pathway, Focal adhesion, cell cycle, tricarboxylic acid cycle (TCA), TGF beta signaling pathway, WNT signaling pathway, Notch signaling pathway, P53 signaling pathway, insulin signaling pathway, calcium signaling pathway, Interleukin-19, Interleukin-20, Interleukin-22, Interleukin-24, IL7, histone deacetylase (HDAC), PKC signaling pathway, RAR pathway, adenylate cyclase signaling pathway, histone methyltransferase (HMT) inhibitors, DNA methyltransferase (DNMT) inhibitors and histone demethylase inhibitors.

In an embodiment, the regulation of the NOD-like receptor signaling pathway is performed by regulating an expression of a gene or protein target selected from the group consisting of NAIP, IL, CXCL12, NODI, TAB3, CARD6, CXCL2, CXCL1, CXCL3, CARD8, CARD9, CASP1, CASP12, CASP4, CASP5, NFKB1, TMEM173, TNF, NFKBIB, NOD2, PYDC1, PYCARD, TAB1, TAB2, TNF, TLR4, NLRP1, NLRP12, NLRP3, NLRP6, MCU, RIPK3, RHOA, TAK1, BIRC2, ATG16L1, ATG5, ATG12, TANK and a combination thereof.

In an embodiment, the regulation of the Focal adhesion pathway is performed by regulating an expression of a gene or protein target selected from the group consisting of TNXB, RAPGEF1, ITGB8, SRC, THBS1, ITGA3, VCL, CAPN2, FLT4, FLT1, ITGA3, ITGB1, ITGB3, ITGB5, ITGB6, ITGB7, ITGA1, ITGA10, ITGA11, ITGA2, ITGA2B, ITGA5, ITGA6, ITGA7, ITGA8, ITGA9, ITGAV, PDRVG, PDGFA, PDGFB, PDGFC, PDGFD, PDGFRA, PDGFRB, BIRC3, BIRC2, BCL2, DOCK1, FN1, HGF, EGF, EGFR, IGF1, IGF1R, VEGFA, VEGFB, VEGFC, CTNNB1 and a combination thereof.

In an embodiment, the regulation of the cell cycle is performed by regulating an expression of a gene or protein target selected from the group consisting of MAD2L1, BUB1, ORC1, ORC2, ORC3, ORC4, ORC5, ORC6, ATM, ATR, CCNA1, CCNA2, CCNB1, CCNB2, CCNB3, CCND1, CCND2, SMAD2, SMAD3, SMAD4, E2F2, E2F3, E2F4, E2F5, EP300, FZR1, GADD45A, GADD45B, STAG1, STAG2, CDC14A, CDC14B, CDC20, CDC25A, CDC25B, MYC, SMC3, CDC16, YWHAH, YWHAB, YWHAQ, YWHAE, YWHAG, YWHAZ and a combination thereof.

In an embodiment, the regulation of the tricarboxylic acid cycle is performed by regulating an expression of a gene or protein target selected from the group consisting of IDH3G, IDH3B, MDH2, SDHB, OGDH, MDH1, OGDHL, SUCLG1, SUCLG2, SUCLA2, SDHA, SDHB, SDHC, PDHA1, PDHB, ACLY and a combination thereof.

In an embodiment, the regulation of the TGF beta signaling pathway is performed by regulating an expression of a gene or protein target selected from the group consisting of ACVR1C, THBS1, FST, TGFB1, TGFBR1, TGFBR2, TGFBR3, BMP4, RUNX3, RUNX2, CREBBP, IFNG, HRAS, FOS, TGFB2, TGFB3, ACVRL1, FOXO3, MTOR, KRAS, CREB1, ATF1, ATF2, ATF4, AKT1, AKT2, AKT3, HNF4A, HNF4G, PIK3R3 and a combination thereof.

In an embodiment, the regulation of the WNT signaling pathway is performed by regulating an expression of a gene or protein target selected from the group consisting of PRKCA, WNT7B, PRICKLE1, LRP6, CTNNB1, FZD4, CCND2, PRICK, WNT5A, WNT1, WNT10A, WNT11, WNT9A, WNT9B, WNT3, WNT4B and a combination thereof.

In an embodiment, the regulation of the Notch signaling pathway is performed by regulating an expression of a gene or protein target selected from the group consisting of CIR1, KAT2B, MAML2, PSEN2, DVL2, RFNG, SNW1, DLL4, DTX3, DLL3, DLL1, DTX1, DTX2, CREBBP, CTBP1, CTBP2, JAG1, JAG2, NOTCH1, NOTCH2, NOTCH3, NOTCH4, PSEN1, PSEN2 and a combination thereof.

In an embodiment, the regulation of the P53 signaling pathway is performed by regulating an expression of a gene or protein target selected from the group consisting of CCNG2, SIAH1, BBC3, TP53AIP1, TP53, SETD7, ATF3, CCNA2, CDK2, CCNG1, CHEK1, PRKCD, KAT2B, PRL23, PPP2CA and a combination thereof.

In an embodiment, the regulation of the calcium signaling pathway is performed by regulating an expression of a gene or protein target selected from the group consisting of RYR1, RYR2, RYR3, ESR1, AR (androgen receptor), KDR (kinase insert domain receptor), VDR (vitamin D receptor), ITPR1, ITPR2, ITPR3, PDE1A, PDE1B, PDE1C, PRKCA, PRKCD, PRKCE, PRKCG and a combination thereof.

In an embodiment, the regulation of the insulin signaling pathway is performed by regulating an expression of a gene or protein target selected from the group consisting of RAPGEF1, PHKG1, PYGL, TRIP10, INS, INSR, IRS1, PDPK1, PIK3CA, HRAS, GRB2, PTPN1, PTPN11 and a combination thereof.

In an embodiment, the regulation of the PKC signaling pathway is performed by regulating an expression of a gene or protein target selected from the group consisting of PRKCA, PRKCB, PRKDC, PRKCZ, PRKCE, PRKCG, PRKCD, PRKCH, PRKCI, PRKCQ, PRKD1, SLC9A5, MAPK3, MAPK9, MAPK8, MAPK1 and a combination thereof.

In an embodiment, the regulation of the RAR pathway is performed by regulating an expression of a gene or protein target selected from the group consisting of RARA, RARS, RARB, RARG, RXRA, RXRG, FAM120B, NCOA1, NCOR2 and a combination thereof.

In an embodiment, the regulation of HDAC is performed by regulating an expression of a gene or protein target selected from the group consisting of HDAC1, HDAC2, HDAC3, HDAC4, HDAC5, HDAC6, HDAC7, HDAC8, HDAC9, HDAC10, HDAC11 and a combination thereof.

In an embodiment, the regulation of adenylate cyclase signaling pathway is performed by regulating an expression of a gene or protein target selected from the group consisting of PRKAR1A, ADCY10, ADCYAP1, ADCY1, ADCY2, ADCY6, ADCY3, GNAI1, GNAL, GNAT3, PRKACA, PRKAR2B, PRKACB, PRKAR1B, PRKACG, CDKN1B, PRKAR2A, NCAM1, CDKN1A (cyclin dependent kinase inhibitor 1A) and a combination thereof.

In an embodiment, the regulation of HMT is performed by regulating an expression of a gene or protein target selected from the group consisting of HNMT, DNMT1, KMT2A, EHMT2, EHMT1, KMT2A, DOT1L, EZH2, SETD7, DNMT3B, DNMT3A, SETDB1, SETD2 and a combination thereof.

In an embodiment, the regulation of DNMT is performed by regulating an expression of a gene or protein target selected from the group consisting of DNMT1, DNMT3B, DNMT3A, CDKN2A, CDKN2B, EHMT2, EHMT1, DNMT3L, CDH1, PARP1, MBD2 and a combination thereof.

In an embodiment, the regulation of histone demethylase is performed by regulating an expression of a gene or protein target selected from the group consisting of KDM1A, KDM4A, KDM5A, KDM5B, KDM2A, KDM5C, KDM4B, KDM4C, KDM5D, KDM4D, KDM1B, HISTIH3A, HIST4H4, HIST2H3C, HAT1, HIST1H4C, HIST1H4F, HIST1H4J, HIST1H2AE, HIST1H2BB, CLOCK, NOCA1 and a combination thereof.

In an embodiment, the method is performed by a small molecule compound combination, a cytokine or recombinant protein combination, gene editing, transgenic technology or a combination thereof.

In an embodiment, the small molecule compound combination is a histone deacetylase inhibitor, a TGF-β receptor inhibitor, a PKC inhibitor, a WNT/β-catenin agonist, a cAMP agonist, a RAR agonist, a ROCK inhibitor, a JNK inhibitor, a DNMT inhibitor, a HMT inhibitor, a histone demethylase inhibitor, a JAK-STAT inhibitor or a combination thereof.

In an embodiment, the histone deacetylase inhibitor is selected from the group consisting of sodium phenylbutyrate, butyrate, sodium butyrate, VPA, Scriptaid, Apicidin, LBH-589(Panobinostat), MS-275, SAHA(Vorinostat), Trichostatin(TSA), Psammaplin A, splitomicin, SRT1720, resveratrol, Sirtinol, APHA, CI-994, Depudecin, FK-228, HC-Toxin, ITF-2357(Givinostat), Chidamide, RGFP 966, PHOB, BG45, Nexturastat A, TMP269, CAY10603, MGCD-0103, Niltubacin, PXD-101 (Belinostat), Pyroxamide, Tubacin, EX-527, BATCP, Cambinol, MOCPAC, PTACH, MC1568, NCH51, TC-H106 and a combination thereof.

In an embodiment, the TGF-β receptor inhibitor is selected from the group consisting of 616452, LY2109761, Pirfenidone, Repsox(E-616452), SB431542, A77-01, A8301, GW788388, ITD-1, SD208, SB525334, LY364947, ASP3029, D4476, SB505124 and a combination thereof.

In an embodiment, the PKC inhibitor is selected from the group consisting of Go6983, Go6976, Bisindolylmaleimide I (GF109203X) and a combination thereof.

In an embodiment, the WNT/β-catenin agonist is selected from the group consisting of MAY-262611, CHIR98014, CHIR99021, LiCl, Li₂CO₃, TD114-2, AZD2858, AZD1080, BIO, Kenpaullone, TWS119, LY2090314, CBM1078, SB216763, AR-A014418 and a combination thereof.

In an embodiment, the cAMP agonist is selected from the group consisting of Forskolin, IBMX, Prostaglandin E2 (PGE2), NKH477, 8-pCPT-2'-O-Me-cAMP, GSK256066, Apremilast(CC-10004), Roflumilast, Cilomilast, Rolipram, Milrinone, 8-Bromo-cAMP, Dibutyryl-Camp, Sp-8-Br-cAMPs and a combination thereof.

In an embodiment, the RAR agonist is selected from the group consisting of TTNPB, Bexarotene, Ch55, Tamibarotene, Retinol, AM580, ATRA, Vitamin A and its derivatives, 13-cis retinoic acid (RA) and a combination thereof.

In an embodiment, the ROCK inhibitor is selected from the group consisting of Y-27632, Y-27632 2HCl, Thiazovivin, Ripasudil(K-115), Fasudil, GSK429286A, RKI-1447, PKI-1313 and a combination thereof.

In an embodiment, the JNK inhibitor is selected from the group consisting of SP600125, JNK Inhibitor IX, AS601245, AS602801, JNK-IN-8 and a combination thereof.

In an embodiment, the DNMT inhibitor is selected from the group consisting of RG108, Thioguanine, 5-Aza-2'-deoxycytidine (Decitabine), SGI-1027, Zebularine, 5-Azacytidine (AZA) and a combination thereof.

In an embodiment, the HMT inhibitor is selected from the group consisting of EPZ004777, EPZ5676, GSK503, BIX 01294, SGC 0946 and a combination thereof.

In an embodiment, the histone demethylase inhibitor is selected from the group consisting of parnate(tranylcypromine), Tranylcypromine(2-PCPA)HCl, SP2509, 4SC-202, ORY-1001(RG-6016), GSKJ1, GSK-LSD1 and a combination thereof.

In an embodiment, the JAK-STAT inhibitor is selected from the group consisting of STAT5-IN-1, JAK3-IN-1, JAK3-IN-7, WP1066, Homoharringtonine, Pyridone 6, Artesunate, ruxolitinib, SH-4-54, Baricitinib, Ruxolitinib phosphate, AG-490, Baricitinib phosphate, SAR-20347, CYT387 Mesylate, AS1517499, Peficitinib, Ruxolitinib sulfate, NSC 74859, Stattic, Tofacitinib citrate, Pimozide, Oclacitinib maleate, Ruxolitinib, S-enantiomer, SB1317, Niclosamide, Scutellarin, Solcitinib, Mogrol, Nifuroxazide, TG101348(SAR302503), AG-1478 (Tyrphostin AG-1478) (EGFR inhibitor), KX2-391 (Src inhibitor), PKI-402 (PI3Kα/β/γ/δ and mTOR inhibitor), NSC 74859 (S3I-201) (STAT3inhibitor), Fludarabine (Fludara) (STAT-1 inhibitor), U0126-EtOH (UO126 EtOH) (MEK1 and MEK2 inhibitor), SGI-1776 free base (Pim1,Pim2 and Pim3 inhibitor), Sorafenib (Nexavar) (VEGFR, PDGFR, c-Raf and B-Raf inhibitor), PLX-4720(B-RafV600E and c-Raf-1Y340D/Y341D inhibitor) and a combination thereof.

In an embodiment, the cytokine or recombinant protein combination comprises PDGFAA, PDGFAB, BMP4, IGF1, bFGF, EGF, VEGF, insulin, Activin A, TGF-beta1, Noggin, BMP-2, Shh, IL-6, CXCL10, CXCL12, CXCL2, HGF, IFN gamma, IL-2, IL-6R alpha, IL-2R alpha, TNF-alpha, TNF-beta, TPO, IGF2, IGFBP5, IGFBP6, IGFBP4, IGFBP7, IGFBP9, PDGF-BB, MMP3, GDF11 and TIMP2.

In an embodiment, the gene editing involves the use of crispr/cas9 and TALEN gene editing to upregulate or knock out a gene or protein target involved in the JAK-STAT signaling pathway, such as STAT5A.

In an embodiment, the transgenic technique involves the use of lentivirus or retrovirus to overexpress or inhibit a gene or protein target in the JAK-STAT signaling pathway, such as STAT5A.

In an embodiment, in the regenerative fibroblasts prepared by the above method, the JAK-STAT signaling pathway is inhibited, where the gene or protein target involved therein that experiences low expression or inhibited expression is SOS1, STAT5B, JAK1, JAK3, SOCS3, IL6ST, STAT1, STAT2, STAT3, STAT4, STAT6, STAT5A, IRF9, IL6, IL6R, IL2, IL2A, IL2B, PRKCD, CXCL12, CXCR4, JAK2, IL15RA, IL20RB, GHR, CXCL2, PRLR or a combination thereof.

In an embodiment, the regenerative fibroblasts prepared by the above method suffer inhibition of the NOD-like receptor, and/or inhibition of the TGF-β receptor signaling pathway, and/or down-regulation of the insulin signaling pathway, and/or up-regulation of the WNT signaling pathway, and/or down-regulation of the notch signaling pathway and/or down-regulation of the p53 signaling pathway.

In an embodiment, the regenerative fibroblasts are derived from normal fibroblasts, where the normal fibroblasts are derived from connective tissues (such as blood, skin, bone marrow and heart) of mammals (such as humans, monkeys, mice and pigs).

In an embodiment, the regenerative fibroblasts are prepared by treating normal fibroblasts with a combination of small molecule compounds, where the combination of small molecule compounds comprises at least one of a Jak-Stat inhibitor, a WNT/β-catenin agonist, a histone deacetylase inhibitor and a cAMP agonist.

In an embodiment, the regenerative fibroblasts are prepared in the presence of at least one of a RAR agonist, a DNMT inhibitor, a HMT inhibitor, a histone demethylase inhibitor, ascorbate, a JNK inhibitor, a PKC inhibitor, a ROCK inhibitor and a TGF-β inhibitor.

In an embodiment, the regenerative fibroblasts are prepared in a stagewise manner respectively using a first composition and a second composition, where the first composition consists of a WNT/β-catenin agonist, a histone deacetylase inhibitor and a cAMP agonist, or consists of a histone deacetylase inhibitor, an inhibitor of TGF-β receptor, a WNT/β-catenin agonist and a cAMP agonist; the second composition comprises a histone deacetylase inhibitor, a TGF-β inhibitor, a WNT/β-catenin agonist, a cAMP agonist, a RAR agonist, a HMT inhibitor, ascorbate, a PKC inhibitor and a ROCK inhibitor.

In an embodiment, the preparation method involves the use of at least one of 0.05-10 mM VPA, 1-15 µM CHIR99021, 0.5-10 µM Repsox, 3-50 µM Forskolin, 1-20 µM Go 6983, 1-25 µM Y-27632, 0.02-1 µM AM580, 0.5-15 µM EPZ004777, 0.2 mM Vc, 0.2-20 µM TTNPB, 1-15 µM 5-Azacytidine and 1-50 µM SP600125. In an embodiment, the normal fibroblasts are first treated with the first composition for 2-10 days, where the first composition consists of 0.05-10 mM of VPA, 1-15 µM of CHIR99021, 0.5-10 µM of Repsox and 3-50 µM of Forskolin, and then the fibroblasts are with the second composition for 4-20 days, where the second composition consists of 0.05-10 mM of VPA, 1-15 µM of CHIR99021, 0.5-10 µM of Repsox, 3-50 µM of Forskolin, 1-20 µM of Go 6983, 1-25 µM of Y-27632, 0.02-1 µM of AM580, 0.5-15 µM of EPZ004777, 0.2 mM of Vc and 0.2-20 µM of TTNPB.

In an embodiment, compared to the normal fibroblasts, the telomere of the regenerative fibroblasts is extended by 1.5 to 12 times, and is close to the cells of the same type in minor individuals in length. Other cells (such as osteoblasts and chondrocytes) derived from the regenerative fibroblasts have longer telomeres and stronger functional activity than similar cells from the same individual.

In an embodiment, products (such as secretion and lysate) derived from the regenerative fibroblasts are applied to the construction of tissue engineering materials and the delaying or reversing of the aging of cells, tissues, organs and the body.

In a second aspect, this application provides an application of the regenerative fibroblasts in the construction of tissue engineering materials and the delaying or reversing of the aging of cells, tissues, organs and the body.

In an embodiment, the regenerative fibroblasts are prepared by knocking out STAT5 gene from normal fibroblasts.

In an embodiment, the regenerative fibroblasts with extended telomere are obtained 3-100 days after the STAT5 gene is knocked out from normal fibroblasts.

In an embodiment, rejuvenated mesenchymal stem cells are prepared by treating mesenchymal stem cells with a combination of small molecule compounds or by gene editing, where the combination of small molecule compounds comprises at least one of a Jak-Stat inhibitor, a WNT/β-catenin agonist, a DNMT inhibitor, a TGF-β inhibitor and a cAMP agonist, and the gene editing is performed by knocking out a gene or protein target in the Jak-Stat signaling pathway (such as STAT5A).

In an embodiment, the mesenchymal stem cells are treated with a combination of 1-15 µM of CHIR99021 and 1-15 µM of 5-Azacytidine (AZA), a combination of 1-15 µM of AZA and 3-50 µM of Forskolin or a combination of 1-15 µM of AZA, 3-50 µM of Forskolin and 1-15 µM of CHIR99021 for 1-28 days for rejuvenation.

In a third aspect, this application provides an application of the regenerative fibroblasts or a culture or lysate thereof in the manufacture of a kit, a drug, a health-care product, food, cosmetics or a medical device.

In a fourth aspect, this application provides use of the regenerative fibroblasts as seed cells or scaffold sources in the preparation of tissue engineering materials, the repairing of damages of mammalian tissues and organs and the repairing of aged and degenerated tissues and organs.

In a fifth aspect, this application provides an application of the regenerative fibroblasts in the medical research or in the preparation of an immunomodulator.

In a sixth aspect, this application provides an application of the regenerative fibroblasts in the *in vitro*/*in vivo* prevention, delaying and reversing of the aging process of mammalian tissues, organs and bodies.

In a seventh aspect, this application provides an application of the regenerative fibroblasts in the reprogramming or rejuvenation of cells, tissues, organs and organisms.

The features of this application are specifically described as follows. By means of regulating the gene or protein targets in the Jak-Stat signaling pathway quantitatively and/or in a timing manner, the cell differentiation, dedifferentiation, transdifferentiation, rejuvenation, aging and apoptosis can be regulated and the aging process can be reversed to extend the lifespan of the body, where the regulation of the gene or protein targets in the Jak-Stat signaling pathway is performed using a combination of small molecule compounds, a cytokine combination, a recombinant protein combination, gene editing or transgenic technique. The gene or protein targets are selected from the group consisting of CXCL2, SOS1, STAT5B, JAK1, JAK3, SOCS3, IL6ST, STAT1, STAT2, STAT3, STAT4, STAT6, STAT5A, IRF9, IL6, IL6R, IL2 (e.g., IL2A and/or IL2B), PRKCD, CXCL12, CXCR4, JAK2, IL15RA, IL20RB, GHR, PRLR and a combination thereof. The combination of small molecule compounds includes at least one of a Jak-Stat inhibitor, a WNT/β-catenin agonist, a histone deacetylase inhibitor, a cAMP agonist, a RAR agonist, a DNMT inhibitor, an HMT inhibitor, a histone demethylase inhibitor, ascorbate, a JNK inhibitor, a PKC inhibitor, a ROCK inhibitor and a TGF-β inhibitor. The cytokine combination or the recombinant protein combination includes PDGFAA, PDGFAB, BMP4, IGF1, bFGF, EGF, VEGF, insulin, Activin A, TGF-beta1, Noggin, BMP-2, Shh (Sonic Hedgehog), IL-6, CXCL10, CXCL12, CXCL2, HGF, IFN gamma, IL-2, IL-6R alpha, IL-2R alpha, TNF-alpha, TNF-beta, TPO, IGF2, IGFBP5, IGFBP6, IGFBP4, IGFBP7, IGFBP9, PDGF-BB, MMP3, GDF11 and TIMP2. The gene editing involves the use of crispr/cas9 gene editing to up-regulate or knock out gene or protein targets in the JAK-STAT signaling pathway, such as STAT5A. The transgenic technique involves the use of lentivirus or retrovirus to overexpress or inhibit gene or protein targets in the JAK-STAT signaling pathway, such as STAT5A.

This application employs a combination of small molecule compounds to inhibit the gene or protein targets in the JAK-STAT signaling pathway (such as STAT5A and JAK1) in fibroblasts to prepare regenerative fibroblasts, in which the JAK-STAT signaling pathway is inhibited. Moreover, the regenerative fibroblasts also experience inhibition of NOD-like receptor signaling pathway, inhibition of TGF beta receptor signaling pathway, down-regulation of the insulin signaling pathway, up-regulation of the WNT signaling pathway, down-regulation of the notch signaling pathway, down-regulation of the p53 signaling pathway or a combination thereof. Compared to the normal fibroblasts, the telomere of the regenerative fibroblasts is extended by 1.5 to 12 times, and is close to the cells of the same type in minor individuals in length. Other cells (such as osteoblasts and chondrocytes) derived from the regenerative fibroblasts have longer telomeres and stronger functional activity than similar cells from the same individual. The regenerative fibroblasts and the secretion and lysate derived therefrom can be applied to the construction of tissue engineering materials and the delaying or reversing of the aging of cells, tissues, organs and bodies.

This application employs the quantitative and/or timing regulation of gene or protein targets in the Jak-Stat signaling pathway to regulate cell differentiation, dedifferentiation, transdifferentiation, rejuvenation, aging and apoptosis, and reverse the aging process to prolong the lifespan, which can promote the transdifferentiation among different types of cells, facilitate the preparation of different types of rejuvenated cells (such as rejuvenated mesenchymal stem cells and super fibroblasts) and promote cell senescence and apoptosis. The rejuvenated mesenchymal stem cells prepared by the method of the invention and the secretion and lysate thereof can be applied in the *in vitro*/*in vivo* prevention, delaying and reversing of the aging of mammalian tissues, organs and bodies, the reprogramming of the cells, tissues, organs and bodies and the repairing of the injured, aged and degenerated mammalian tissues and organs, and can also be used as seed cells and scaffold sources for the tissue engineering materials.

The mechanism of the invention is described as follows. The expression of gene or protein targets in the Jak-Stat signaling pathway in cells is regulated quantitatively and/or in a timing manner to differently regulate the metabolic pathways, changing the cell state of the target cell to allow it to transform into other cells or to possess different cell characteristics.

Compared to the prior art, this application has the following beneficial effects.

Compared to the fibroblasts derived from the same donor or the fibroblasts derived from a different donor at the same age, the regenerative fibroblasts are free of tumorigenicity. The regenerative fibroblasts are characterized by the changes in epigenetics, and/or changes in the expression of senescence-related genes, and/or the extension of cell telomeres, and/or the acceleration of cell proliferation, and/or the ability to perform long-term stable passage. In addition, the regenerative fibroblasts and products thereof can reverse the aging of the mammalian organisms and prolong the lifespan. The quantitative and/or timing regulation of gene or protein targets in the Jak-Stat signaling pathway provided herein can systematically regulate the cell differentiation, dedifferentiation, transdifferentiation, rejuvenation, aging and apoptosis, and the resulting regenerative fibroblasts and products therefrom can be used in the *vitro*/*in vivo* prevention, delaying and reversing of the aging of mammalian tissues, organs and bodies, the reprogramming of the cells, tissues, organs and bodies and the repairing of the injured, aged and degenerated mammalian tissues and organs, and can also be used as seed cells and scaffold sources for the tissue engineering materials.

### BRIEF DESCRIPTION OF THE DRAWINGS

Fig. 1 schematically shows the preparation of regenerative fibroblasts (rFib) from fibroblasts (Fib).
Fig. 2 schematically illustrates that the rFib is more rejuvenated when compared to Fib and bMSC.
Fig. 3 illustrates that the rFib is superior to aged bMSC in osteogenic and chondrogenic differentiation abilities, and is free of tumorigenicity.
Fig. 4 depicts the *in vitro* immunomodulatory activity of rFib.
Fig. 5 shows the *in vivo* immunomodulatory activity of rFib.
Fig. 6 shows that the rFib has the ability to repair bone defects regardless of donor age.
Fig. 7 shows results of *in vivo* cartilage repair experiment.
Fig. 8 shows that the inhibition of STAT5 gene can rejuvenate Fib to possess multiple differentiation potentials.
Fig. 9 shows expression levels of STAT5A and H3K9me after the STAT5 gene is knocked out.
Fig. 10 illustrates measurement results of rejuvenation degree and differentiation ability of another STAT5-knockout Fib from a 62-year-old donor.
Fig. 11 depicts β-galactosidase staining results of MSCs treated with various compound combinations for 3 days, where 55Y: 55 years old; 82Y: 82 years old; and Y: age of donor.
Fig. 12 demonstrates that rFib enables the NOD/SCID mice to have prolonged lifespan and improved bone density.
Fig. 13 shows that the rFibs are distributed in multiple organs of mice and can differentiate into functional cells.
Fig. 14 illustrates the improvement of rFib on bone density of aged mice with osteoporosis.
Fig. 15 shows the rFib culture can significantly promote the skin healing, where the mice injected with rFib culture were almost completely healed 12 days after modeling.
Fig. 16 shows the improvement of rFib on the lower extremity ischemia in mice.
Fig. 17 illustrates that the treatment with Mix Y can inhibit the expression of STAT5 and STAT3 (A-B), down-regulate the expression of CDKN1A (C) and extend the telomere, rejuvenating the cells.
Fig. 18 demonstrates that the treatment with Mix Pn can down-regulate the expression of STAT5 (A) and inhibit the expression of ATF3, CDKN1A, GADD45B and IL6 (B-E), achieving the rejuvenation of Fib.
Fig. 19 illustrates that the treatment with Mix Y-Mix Pn2 can inhibit the expression of JAK1 (A) and extend the telomere (B) in Fib.
Fig. 20 shows the transdifferentiation from Fib into neurons, where A: staining of Tuj 1 in neurons transdifferentiated from Fib; B: expression level of Nestin.
Fig. 21 shows that under the action of a small molecular combination, the expression of STAT5 gene is up-regulated in the differentiation of ES into neurons.
Fig. 22 shows modules enriched in the KEGG pathway.

### DETAILED DESCRIPTION OF EMBODIMENTS

### Example 1 Preparation and characterization of regenerative fibroblasts

1. Human skin fibroblasts were inoculated into a 6-well plate and cultured in a Fib culture medium for 24 hours.
2. The cell culture medium was replaced with an induction culture medium containing a cocktail (Mix V) of small molecules, and then the medium was replaced every 2 days.
3. After cultured in the induction culture medium containing Mix V for 5 days, the skin fibroblasts were transferred to an induction culture medium containing Mix P, and the medium was replaced every 2 days.
4. After cultured in the induction culture medium containing Mix P for 7 days, the skin fibroblasts were transferred to a HG (high glucose)-DMEM containing 10% FBS, 10 ng/mL of bFGF, 100 ng/mL of PDGF-AB and 10 ng/mL of BMP4 or merely containing 10% FBS, or to a rFib medium for culture. After cultured for another 3 days, the cells were subjected to long-term passage and characterization.
5. During the long-term passage, the rFibs were cultured in a MSC basal medium and subcultured when the confluency reached 90%.

The Fib culture medium was a HG-DMEM containing 10% FBS or a commercially-available Fibstar-CO medium (cat. no. FMS003C, HCell).

The Mix V was a HG-DMEM supplemented with 10% FBS, 0.5 mM of VPA, 3 µM of CHIR99021, 1 µM of Repsox and 10 µM of Forskolin.

The Mix P was a HG-DMEM supplemented with 10% FBS or a commercially-available FibGro medium (cat. no. FGS0040, HCell), containing 0.5 mM of VPA, 3 µM of CHIR99021, 1 µM of Repsox, 10 µM of Forskolin, 10 µM of SP600125, 5 µM of Go 6983, 5 µM of Y-27632, 0.05 µM of AM580, 5 µM of EPZ004777, 0.2 mM of Vc and 5 µM of TTNPB.

The MSC basal medium was LG-DMEM supplemented with 10% FBS, a commercially-available complete medium for bone marrow mesenchymal stem cells (cat. no. HUXMA-90011, Cyagen) or a commercially-available rFib medium (cat. no. CRM0016-01, HCell).

It should be noted that unless otherwise specified, the cells used in the examples are derived from human.

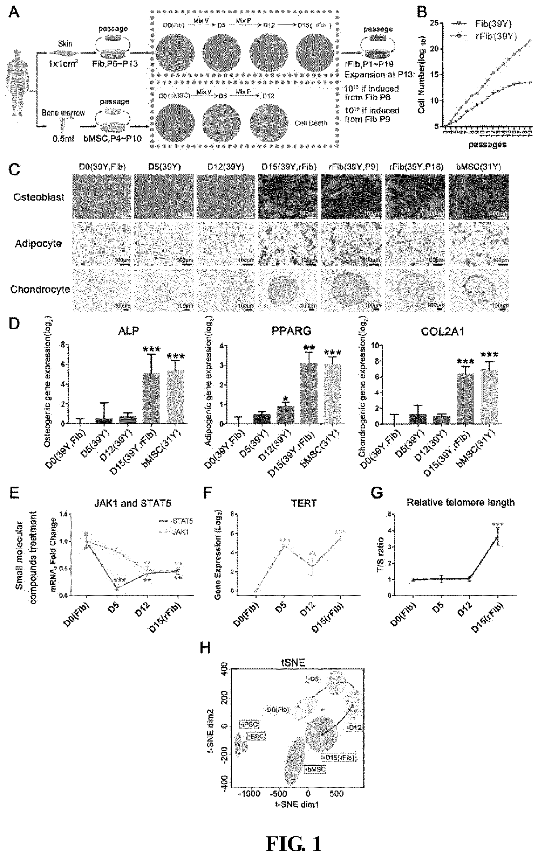

Fig. 1A schematically showed the preparation of rFib from Fib, from which it can be seen that the rFib derived from the nearly aged Fib (passage 13) had considerable potential for expansion (can experience another 19 passages), while the same treatment process induced the death of bone marrow mesenchymal stem cells.

Fig. 1B illustrated growth curves of rFib and its homologous Fib during the long-term expansion, in which rFib presented a better growth rate than the homologous Fib.

The differentiation potentials of Fib and rFib were analyzed by histochemical assay and the results were displayed in Fig. 1C, where the parental Fib was a passage 8 Fib derived from a 39-year-old volunteer. The cell was tested for its osteogenic, adipogenic and chondrogenic potentials respectively on day 0 (Fib, before treatment), day 5, day 12 and day 15 (rFib), and before test, the cell was subjected to several expansions. After 21 days of the induction, the differentiated osteoblasts, adipocytes and chondrocytes were identified by staining respectively with alizarin red, oil red and alician blue. Moreover, the staining results also demonstrated that after passaged several times (9 and 16), the rFib still maintained desirable differentiation potentials.

The expression levels of ALP (14 days after induced osteogenic differentiation), COL2A1 (14 days after induced chondrogenic differentiation) and PPARG (21 days after induced adipogenic differentiation) were analyzed by q-RT-PCR (Fig. 1D), and the results revealed that similar to young bMSCs, the rFibs can also highly express genes related to trilineage differentiation (**p*<0.05, ***p*<0.01, ****p*<0.001, n≥3, the significance analysis was performed by comparison with D0(Fib)).

Figs. 1E-G showed changes of expression levels of JAK1, STAT5 and telomerase (TERT) and change of telomere length over time after fibroblasts were treated with small molecular compounds, from which it can be observed that the treatment brought a decrease in the expression levels of JAK1 and STAT5; the telomerase experienced high expression on the 5^{th} day of the treatment; and the telomere was significantly extended on the 15^{th} day of the treatment.

The transcriptome data of Fib, rFib, iPSC, ESC and bMSC was subjected to cluster analysis (Fig. 1H), and the results demonstrated that the rFib was more similar to Fib and bMSC relative to iPSC and ESC, which indicated that the rFib simultaneously possessed the characteristics of fibroblasts and mesenchymal stem cells. Moreover, the rFibs were safe and had no tumorigenicity.

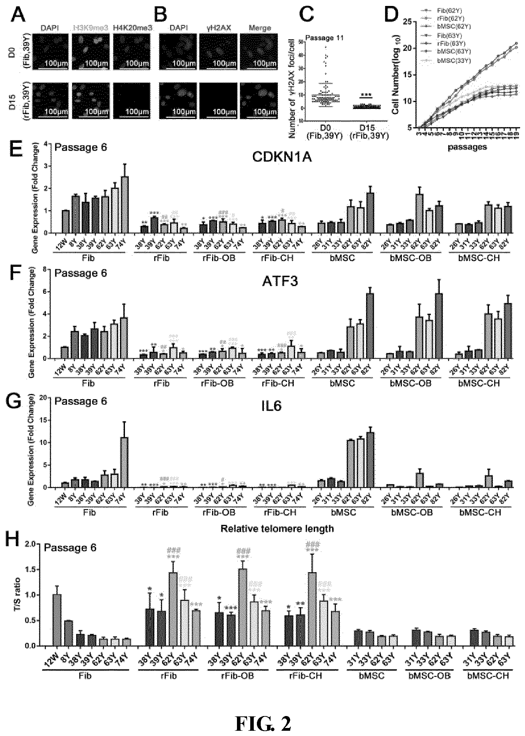

As shown in Fig. 2, the rFib possessed rejuvenation-related characteristics compared to Fib and bMSC.

Fig. 2A displayed immunofluorescence staining results of senescence markers H3K9me3 and H4K20me3 in Fib (D0, passage 11 parental Fib) and rFib (D15), from which it can be concluded that the rFib had significantly reduced H4K20me3 compared with the homologous Fib.

Figs. 2B-C respectively displayed immunofluorescence staining result and quantification of senescence marker γH2AX in Fib (D0, passage 11 parental Fib) and rFib (D15), from which it can be concluded that the rFib had significantly reduced γH2AX compared with the homologous Fib.

Fig. 2D showed growth curves of Fib, rFib and bMSC respectively derived from two old volunteers during the long-term subculture, where the cells from the same donor were indicated by the same color. As illustrated in this figure, the rFib grew significantly faster than bMSC and Fib from the same donor, and the growth rate of the rFib from the elderly donor was even higher than that of the bMSC from the young donor (33 years old).

Expression levels of several senescence markers (CDKN1A, ATF3 and IL6) in Fib, rFib, rFib-OB, rFib-CH, bMSC, bMSC-OB and bMSC-CH were detected by q-RT-PCR (Figs. 2E-G, where 12W indicated the skin cells of the aborted fetus at 12^{th} week of pregnancy). It was apparent that the rFibs and the osteoblasts and chondrocytes derived therefrom all experienced significantly reduced expression of such senescence markers.

The relative telomere length of Fib, rFib, rFib-OB, rFib-CH, bMSC, bMSC-OB and bMSC-CH was detected by q-RT-PCR and expressed by T/S ratio (Fig. 2H).

In Figs. 2E-H, the cells from the same donor were indicated by the same color; * indicated significant difference when compared to homologous Fib; # indicated significant difference when compared to corresponding bMSC, bMSC-OB (osteoblasts derived from bMSC) and bMSC-CH (chondrocytes derived from bMSC); **p*<0.05, ***p*<0.01, ****p*<0.001, *#p<0.05, ##p*<0.01, *###p*<0.001, n=3.

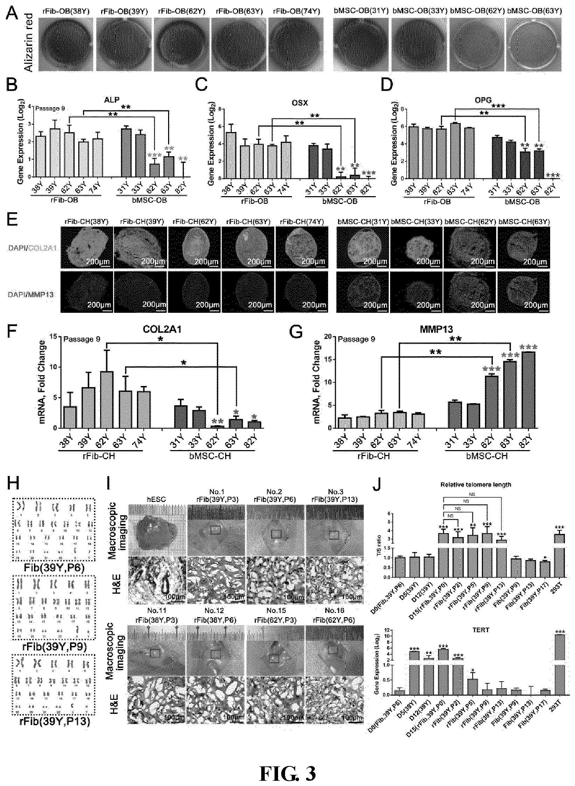

Fig. 3 revealed that the osteogenic and chondrogenic differentiation abilities of aged bMSC were inferior to the rFib, and the rFib was free of tumorigenicity.

After experiencing the osteogenic differentiation, the bMSC and rFib from donors of different ages were subjected to alizarin red staining, and the results were shown in Fig. 3A. It can be found that the osteogenic differentiation potential of the bMSC from an elderly donor was greatly declined, while the rFib of the same elderly donor still maintained a desirable osteogenic differentiation ability.

Figs. 3B-D showed expression levels of marker genes in osteoblasts respectively derived from rFib and bMSC from volunteers of different ages, where the expression levels of ALP, OSX and OPG were all significantly higher in the rFib.

COL2A1 and MMP13 in chondroblasts respectively derived from rFib and bMSC from volunteers of different ages were subjected to immunohistochemical staining, and the results were exhibited in Fig. 3E. The chondroblasts derived from bMSC from an elderly donor involved low expression of COL2A1 and high expression of MMP13, while chondroblasts derived from rFib from an elderly donor were similar to those derived from young rFib and bMSC in the expression of COL2A1 and MMP13.

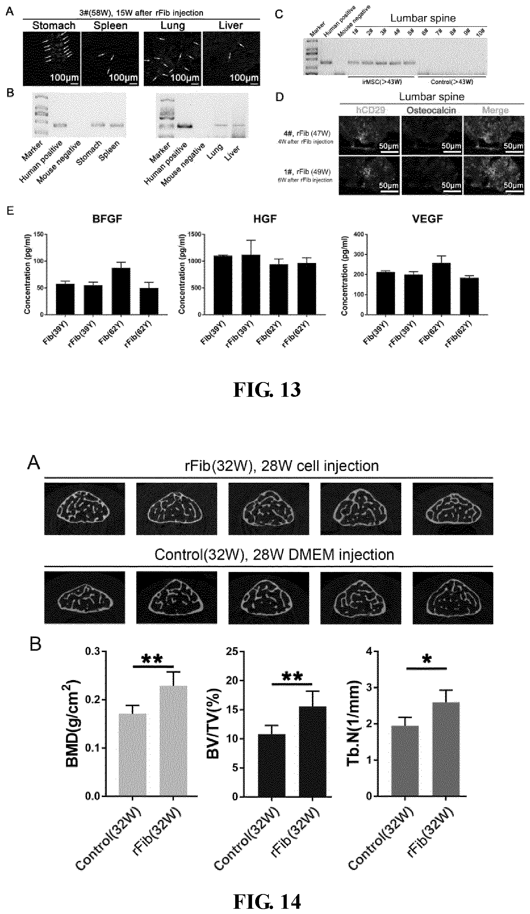

Further, the expression levels of COL2A1 and MMP13 in chondroblasts respectively derived from rFib and bMSC from volunteers of different ages were analyzed by q-RT-PCR (Figs. 3F-G), and the results were consistent with the staining results presented in Fig. 13.

Fig. 3H depicted the karyotype analysis results of passage 9 rFib, passage 13 rFib and their homologous passage 6 Fib, from which it can be observed that the karyotype of rFib remained consistent with its parental Fib even after long-term passage.

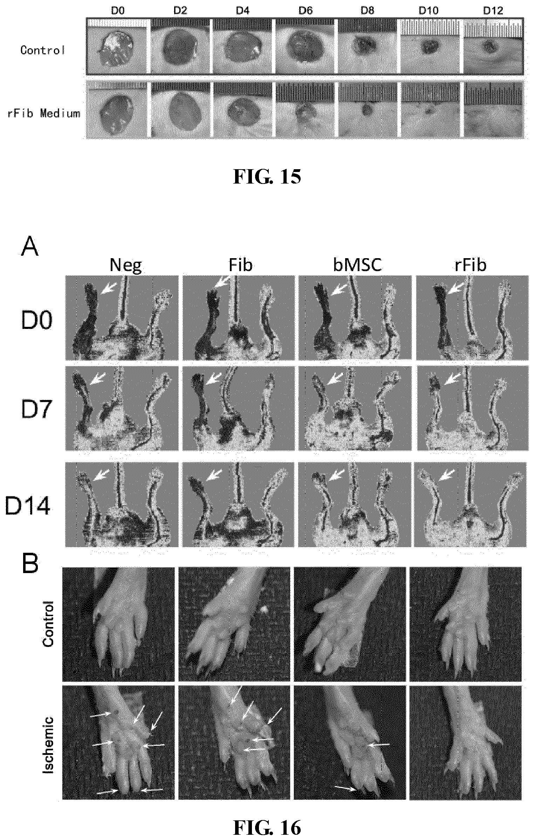

The tumorigenicity of rFib was tested by macroscopic imaging and H&E staining, and the results were shown in Fig. 3I, where human embryonic stem cells (hESC) were used as positive control. As illustrated in Fig. 16, the subcutaneous transplantation of hESC resulted in the occurrence of teratomas (with obvious three-germ structure) in the NOD/SCID mice, while no tumors were found in mice transplanted with rFib.

Fig. 3J illustrated the relative telomere length and expression level of telomerase in Fib and rFib, where compared with its homologous Fib, the rFib had significantly longer telomeres and the telomere length can still be maintained after induction. However, in the rFib, the telomerase only temporarily exhibited high expression during the induction and then returned to a low expression level, which was different from tumor cells (the telomerase experienced persistent high expression), indicating that the rejuvenation process will not bring tumorigenicity in the rFib.

### 6.1 In vitro immunomodulation test

The Fib, rFib and bMSC were treated with mitomycin C for 2.5 hours, digested and counted, and then respectively inoculated to a 24-well plate at 1×10⁵ cells/well. T lymphocytes were stained with carboxyfluorescein succinimidyl ester (CFDA-SE) at 37°C for 30 min and then inoculated to the 24-well plate at 2×10⁵ cells/well. PHA (phytohemagglutinin) was added at a final concentration of 2 µg/mL to stimulate the proliferation of lymphocytes. Three experimental groups (bMSC+T+PHA group, rFib+T+PHA group and Fib+T+PHA group), a positive control group (T+PHA) and a negative control group (T alone) were set, and after co-cultured for 5 days, the T cells in each well were collected and washed with PBS three times. The proliferation of T lymphocytes was examined using anti-CD3, CD4 and CD8 antibodies (BD biosciences) by flow cytometry.

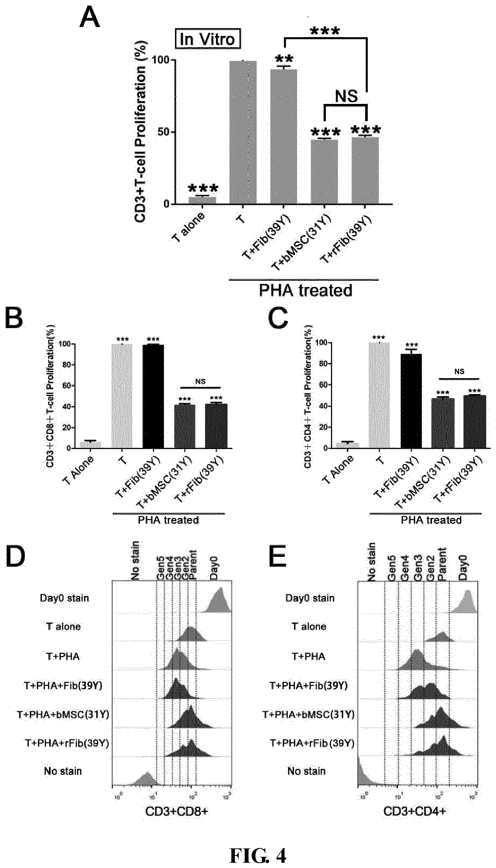

Fig. 4 showed that the rFib had *in vitro* immunomodulatory activity.

Parental Fib, rFib and bMSC were respectively cultured with T cells according to the mixed lymphocyte reaction method, and the proliferation rate of T cells in each group was detected and the results were shown in Fig. 4A (***p*<0.01; ****p*<0.001; n=3; the significance analysis was performed by comparison with the "T+PHA" group). Peripheral blood mononuclear cells (PBMC) from a healthy volunteer were labeled with CFSE. The results demonstrated that the rFib was able to inhibit the proliferation of T cells, while its homologous Fib cannot inhibit the proliferation of T cells, indicating that the rFib had immunomodulatory function.

As shown in Figs. 4B-C, the rFib exhibited the ability to regulate CD3+ CD8+ T cells; and as shown in Figs. 4D-E, the rFib can regulated the proliferation of CD3+ CD4+ T cells.

### 6.2 In vivo immunomodulation test

The bMSC, rFib and Fib were respectively cultured in a 10 cm petri dish at a density of 1×10⁶ for 48 hours, and then the culture medium was collected and filtered with a 0.22 µm filter membrane (Millipore) to remove the cells and cell debris. The resulting filtrate was concentrated by 100 times using an ultrafiltration centrifuge tube.

C57BL/6 mice, aged 8-12 weeks, were injected with concanavalin (diluted in PBS) via tail vein at 25 mg/kg (body weight) to induce acute liver injury, and some C57BL/6 mice were only injected with PBS as control (Han et al., 2014). Each group included 6 mice. 30 min later, the groups were respectively injected with different concentrated mediums and PBS, and the mice were sacrificed 8.5 hours after the injection of concentrated medium. The blood and liver were collected, where the liver was stained with H&E and detected for the content of CD3+ T cells by flow cytometry, and the blood was analyzed for the AST and ALT levels.

The quantification of serum ALT/AST was performed according to the instructions of an ELISA kit (Shanghai Meilian). Three independent replicate samples in each group were tested, and the results were expressed as mean ± SD.

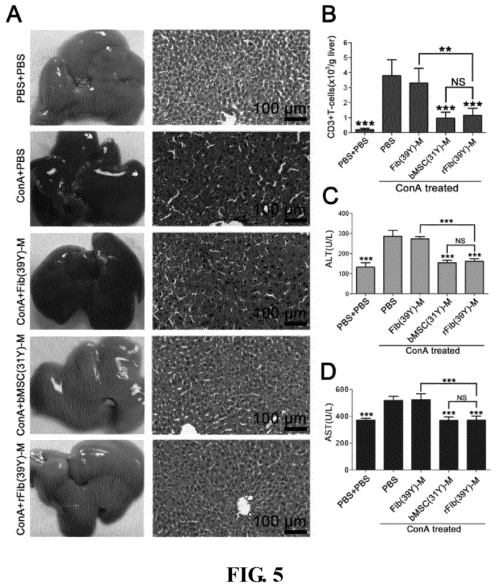

Fig. 5 demonstrated the *in vivo* immunomodulatory activity of rFib.

As shown in Fig. 5A, it can be seen that after treated with the concentrated rFib medium, the C57BL/6 mice suffering concanavalin-induced acute liver injury showed no obvious abnormal symptoms in the liver (such as bleeding and necrosis).

Fig. 5B showed the content of T lymphocytes in liver which was determined 8.5 hours after the tail vein injection of the concentrated medium, where the rFib medium exhibited significant immune regulation ability, similar to the bMSC medium.

As shown in Figs. 5C-D, the blood ALT and AST of the mice treated with rFib culture fluid were close to normal levels, and there were no obvious liver injury symptoms.

### 6.3 PCR of normal genes

The extraction of total RNA was performed as instructed by TRIzol kit (Takara Bio). The reverse transcription of RNA (1.0 µg) into cDNA was performed using Primescript RT kit (Takara Bio). The q-RT-PCR system contained the cDNA as template, a pair of specific primers and SYBR Green, and employed SYBR Premix EX TaqTM II (Takara Bio). Parameters of cycles were set as recommended by the manufacturer (Takara). The relative expression level was normalized using an internal reference (ACTIN). In gPCR, the genomic DNA was used as a template for human-specific primer ACTIN, and Premix Taq (Takara Bio) was adopted.

### Example 2 Repair capability of rFib for bone defects

Under the approval of the ethics committee, NOD/SCID mice, aged 8-10 weeks and weighing 20-24 g were used to create femoral defect models and 5 mice in each group. The model was established as follows. Under the anesthesia of sodium pentobarbital, the skin and subcutaneous tissues of the mice were incised, and blunt separation was performed between the rectus femoris and semitendons to expose enough mid-femur. The operation is performed at the center of the right femur to construct continuous bone defect of 4mm×1mm. The Fib, bMSC and rFib were stained with Hoechst 33342 (Thermo, NucBlue live cell), respectively mixed with Matrigel and transplanted into the defect site at 5×10⁵ cells/mouse.

28 days after transplantation, the mice were sacrificed by injection of a lethal dose of sodium pentobarbital. The thigh of each mouse was bluntly dissected, fixed with 4% PFA and imaged by µCT (SkyScan 1272, Bruker microCT), and the collected data was analyzed.

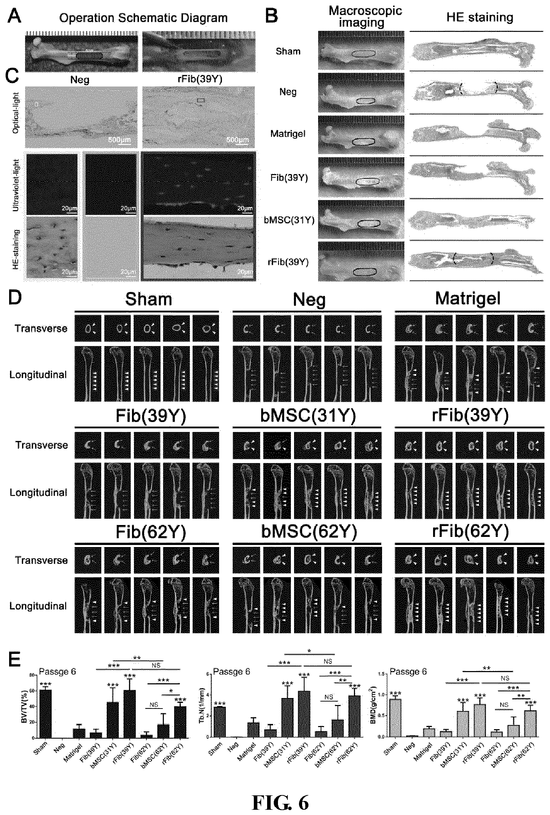

Fig. 6 showed that the bone repair capability of rFib was not limited by age.

Fig. 6A schematically showed the establishment of the femoral defect mouse model. Fig. 6B illustrated femur samples of mice in different groups and H&E staining results thereof, where the bMSC from a 31-year-old volunteer and the rFib from a 39-year-old volunteer both exhibited good repair capability for bone defects.

Fig. 6C illustrated sections of the repair site, in which the rFib was labeled with Hoechst 33342 and can emit blue fluorescence spontaneously. It was clear that the rFib can form new bone at the defect site, and the rFibs were in one-to-one correspondence to the new bone cells in the number and location.

Fig. 6D showed micro-CT results of different experimental groups, from which it can be obtained that even the rFib from an elderly donor (62 years old) possessed repair capability for bone defects, and by contrast, the bone repair capability of the bMSC from an elderly donor (62 years old) was extremely weak. Moreover, the rFib from a young donor (39 years old) and the rFib from an elderly donor (62 years old) had similar bone repair capabilities, which indicated that the repair capability of rFib for bone defects was not limited by the age of donor.

As shown in Fig. 6E, the mice transplanted with the rFib (39 years old) were similar to the mice transplanted with bMSC (31 years old) in BV/TV ratio, Tb.N and BMD. Moreover, the age of the rFib donor will not affect such parameters of mice.

### Example 3 Repair capability of rFib for cartilage defect

### Establishment of articular cartilage defect model and cell transplantation

NOD/SCID mice, weighing 20-24 g and aged 8-10 weeks, were selected to establish a modified articular cartilage model to evaluate the efficacy of rFib (Cheng et al., 2014). The articular cartilage defect (1.5mm×1mm) was made in the trochlear groove of the distal femur with a biopsy punch. Cells (2.5×10⁵ in 35 µL of Matrigel) were labeled with Hoechst 33342 and implanted into the defect site, and the mice implanted with matrigel free of cells were used as control.

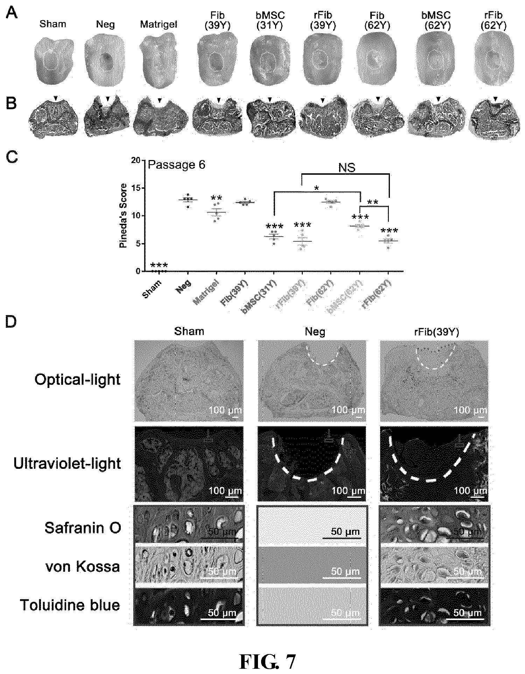

Fig. 7 schematically showed an *in vivo* cartilage repair experiment.

Figs. 7A-B respectively showed cartilaginous tissue and safranin-fast green staining results of a 10 µm section, where the cartilage was stained red and the bone tissue was stained green. It can be seen from these figures that both young rFib (39 years old) and bMSC (31 years old) can repair cartilage defects; the old bMSC (62 years old) can generate new tissues, but failed to form new cartilage; and the rFib from an elderly donor (62 years old) can still form cartilage tissues.

Fig. 7C showed Pineda's score for the cartilage repair in all experimental groups, and the results revealed that the rFib from an elderly donor (62 years old) and the rFib from a young donor (39 years old) had similar cartilage repair capabilities.

The cartilage section demonstrated that the rFib labeled with Hoechst 33342 formed new cartilage tissues, and the newly formed cartilage tissues were similar to normal cartilages in structure (Fig. 7D). Moreover, no abnormal tissues were observed after the implantation of rFib.

### Examples 4-12 Preparation of rFib using a small molecular combination

Examples 4-12 all provided preparation of rFib, but they varied in the used small molecular combination and the treatment time. The obtained rFib was characterized in the way mentioned in Example 1, and the small molecular combinations were listed in Table 1.

**Table 1 Information about the preparation of rFib in Examples 4-12**

| Example | Compound | Concentration | Treatment time (day) | Cell source |
|---|---|---|---|---|
| 4 | VPA | 0.05mM | 9 | Human, monkey, mouse |
| | CHIR99021 | 1µM | | |
| | Repsox | 0.5µM | | |
| | Forskolin | 3µM | | |
| 5 | VPA | 10mM | 9 | Human, monkey, mouse, pig |
| | CHIR99021 | 3µM | | |
| | Repsox | 10µM | | |
| | Forskolin | 10µM | | |
| | SP600125 | 1µM | | |
| | Go 6983 | 5µM | | |
| | Y-27632 | 5µM | | |
| | AM580 | 0.05µM | | |
| | EPZ004777 | 5µM | | |
| | Vc | 0.2 mM | | |
| | TTNPB | 5µM | | |
| 6 | VPA | 0.5mM | 12 | Human, monkey |
| | CHIR99021 | 15µM | | |
| | Repsox | 1µM | | |
| | Forskolin | 50µM | | |
| | SP600125 | 10µM | | |
| | Go 6983 | 20µM | | |
| | Y-27632 | 25µM | | |
| | AM580 | 0.02µM | | |
| | EPZ004777 | 5µM | | |
| | Vc | 0.2mM | | |
| | TTNPB | 0.2µM | | |
| 7 | VPA | 0.5mM | 12 | Human, monkey |
| | CHIR99021 | 3µM | | |
| | Repsox | 1µM | | |
| | Forskolin | 10µM | | |
| | SP600125 | 50µM | | |
| | Go 6983 | 1µM | | |
| | Y-27632 | 1µM | | |
| | AM580 | 1µM | | |
| | EPZ004777 | 0.5µM | | |
| | Vc | 0.2mM | | |
| | TTNPB | 5µM | | |
| 8 | VPA | 0.5mM | 9 | Human, monkey, mouse, pig |
| | CHIR99021 | 3µM | | |
| | Repsox | 1µM | | |
| | Forskolin | 10µM | | |
| | Go 6983 | 5µM | | |
| | Y-27632 | 5µM | | |
| | AM580 | 0.05µM | | |
| | EPZ004777 | 15µM | | |
| | Vc | 0.2mM | | |
| | TTNPB | 5µM | | |
| 9 | CHIR99021 | 2µM | 15 | Human, monkey, mouse, pig |
| | Repsox | 2µM | | |
| | Forskolin | 4µM | | |
| 10 | Ruxolitinib | 0.006µM | 12 | Human |
| 11 | VPA | 0.5mM | 10 | Human, monkey, mouse |
| | CHIR98014 | 3µM | | |
| | Repsox | 1µM | | |
| | Forskolin | 10µM | | |
| | SP600125 | 10µM | | |
| | Go 6983 | 5µM | | |
| | Y-27632 | 5µM | | |
| | AM580 | 0.05µM | | |
| | EPZ004777 | 5µM | | |
| | Vc | 0.2 mM | | |
| | TTNPB | 20µM | | |
| 12 | Ruxolitinib | 0.006µM | 10 | Human, monkey, mouse |
| | S31-201 | 10µM | | |

### Example 13 Preparation of super fibroblasts

1. A CRISPR/Cas9 STAT5a-knockout plasmid was constructed, in which the following plasmids were used (purchased from Cyagen Co., Ltd):
   pLV[2gRNA]-EGFP:T2A:Puro-U6>hSTAT5A[gRNA#4]-U6>hSTAT5A[gRNA#10]; and
   pLV[Exp]-CBh>hCas9:T2A:Hygro.
2. Cells were transfected with viruses as recommended by the manufacturer. On the first day after transfection, the virus-containing medium was replaced with a fresh complete medium, and the incubation was performed at 37°C and 5% CO₂.
3. From the second day after the transfection, the genes carried by the lentivirus began to express and the cells can be continuously cultured to further accumulate the expression products or change the cell phenotype.
4. After expansion, the virus-transfected cells were purified with antibiotics and continuously cultured with HG-DMEM containing 10% FBS for 150 days.

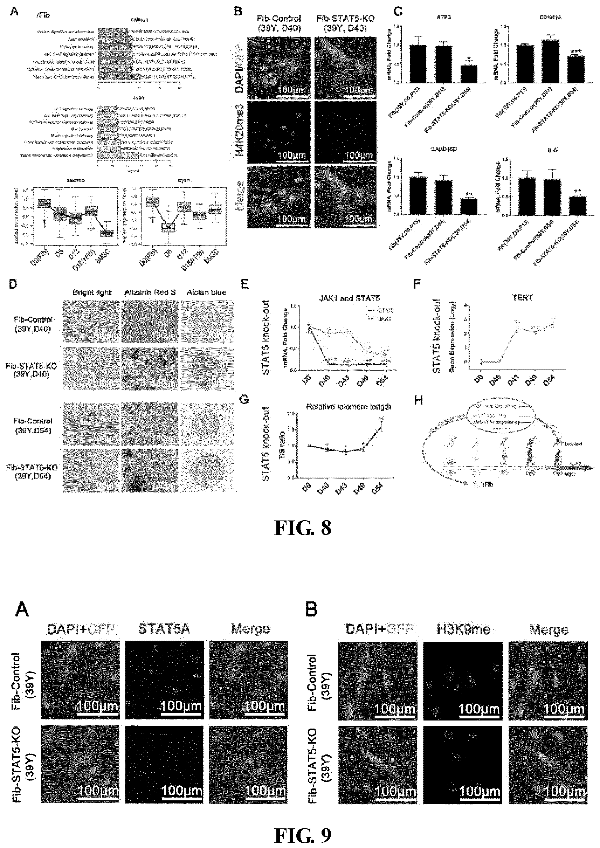

Fig. 8 demonstrated that the inhibition of STAT5 gene can promote the rejuvenation of fibroblasts and allow the fibroblasts to acquire multiple differentiation abilities.

Fig. 8A showed two representative gene modules enriched in the KEGG pathways of rFib and their expression during the induction, where the two representative gene modules were screened based on 12,036 genes identified by WGCNA.

After continuously cultured for 40 days after the knockout of STAT5 gene, the Fib was subjected to immunohistochemical staining, and the results were shown in Fig. 8B. This figure illustrated that compared to the control Fib, the STAT5-KO Fib (Fib with STAT5 gene knocked out) had significantly reduced senescence marker H4K20me3 (the content was positively correlated with the aging degree).

Fig. 8C showed expression levels of four senescence marker genes ATF3, GADD45B, IL6 and CDKN1A (high expression level in aged cells), from which it can be concluded that the STAT5-KO Fib experienced significant reduction in the expression levels of senescence marker genes.

The STAT5-KO Fib exhibited osteogenic (Alizarin Red S staining) and chondrogenic (Alcian Blue staining) capabilities (Fig. 8D).

Figs. 8E-G depicted changes in expression of JAK1, STAT5 and TERT and changes in the relative telomere length after the STAT5 gene was knocked out, where the JAK1 and STAT5 showed declined expression; the telomerase was highly expressed 43 days after the knockout of STAT5 gene; and the telomere length was significantly increased on the 54^{th} day after the knockout of STAT5 gene.

Fig. 8H schematically showed the preparation of rFib from Fib by regulating the Jak-Stat signaling pathway.

Fig. 9 showed influence of STAT5 knockout on STAT5A and H3K9me. Specifically, the STAT5A was no longer expressed after the STAT5 was knocked out (Fig. 9A); and the knockout of STAT5 showed no significant influence on H3K9me (Fig. 9B).

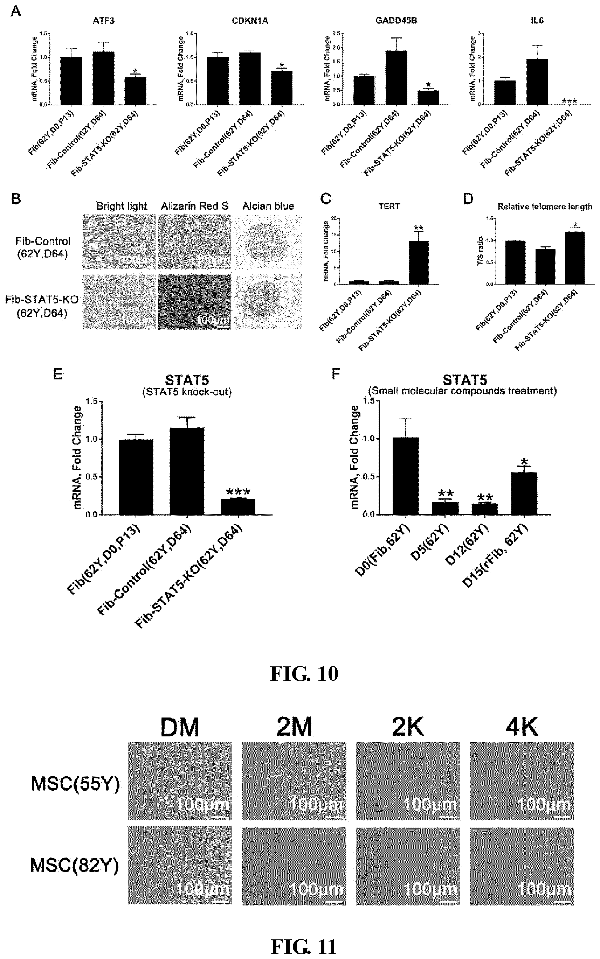

Fig. 10 reflected the detection results of senescence-related indexes and differentiation potentials of another STAT5-KO Fib (derived from a 62-year-old donor).

Specifically, the expression levels of senescence-related genes were shown in Fig. 10A; the measurement results of osteogenic and chondrogenic differentiation potentials were presented in Fig. 10B; the expression level of TERT and the relative telomere length were shown in Figs. 10C-D; the expression level of STAT5 was shown in Fig. 10E; and the expression level of STAT5 in the same cell line treated with Mix V+Mix P system was shown in Fig. 10F.

### Example 14 Rejuvenation of MSCs

1. Bone marrow mesenchymal stem cells (bMSCs) from different donors were respectively cultured in a LG (low glucose)-DMEM containing 10% FBS.
2. After treated with different compound combinations for 3 days, the cells were continuously cultured in the LG-DMEM containing 10% FBS for 3 days and then stained with β-galactosidase.

**Table 2 Compound combinations and treatment time**

| Combination | Compound | Concentration | Treatment time (day) |
|---|---|---|---|
| 2M | CHIR99021 | 3µM | 3 |
| | AZA | 1µM | |
| 2K | CHIR99021 | 3µM | 3 |
| | AZA | 5µM | |
| 4K | CHIR99021 | 1µM | 3 |
| | AZA | 2µM | |
| | Forskolin | 10µM | |

After treated with different compound combinations for 3 days, the MSCs from donors of different ages were subjected to β-galactosidase staining, and the results were shown in Fig. 11.

### Example 15 Extension of lifespan of aged mice through intravenous injection of rFib

Passage 9 Fib and passage 13 rFib from a 39-year-old donor and passage 8 rFib were labeled with Hoechst 33342 and suspended with 200 µL of DMEM, respectively. The cells were injected into naturally aged NOD/SCID mice (aged 43 weeks (approximately corresponding to 86 years of humans), average life span: 36-38 weeks) through tail vein at 10⁶/mouse. The mice in the vehicle group were only injected with 200 µL of DMEM. Tissues and organs were collected for detection after the mice died naturally.

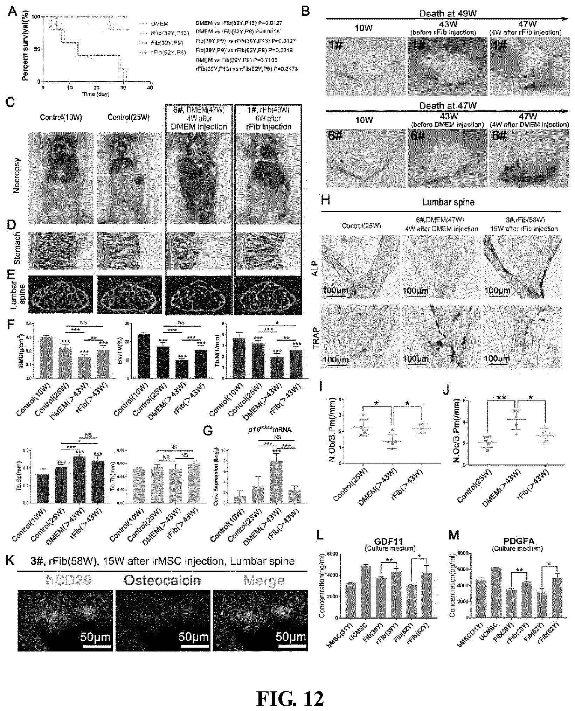

Fig. 12 demonstrated that the rFib can effectively prolong the life span of NOD/SCID mice and improve the bone density.

Specifically, Fig. 12A showed survival curves of the aged mice, where the mice injected with rFib (whether derived from a young donor (39 years old) or an elderly donor (62 years old)) had extended life span. By contrast, even the young Fib had no effect on extending lifespan, and the survival curve of the mice injected with the young Fib was similar to that of the mice in the vehicle group.

The morphological results of the two groups of mice were shown in Fig. 12B, where obvious aging symptoms (messy and dull hair, and humpback) were observed in the mice aged 43 weeks. After four weeks of injection of rFib, the mice's conditions were significantly improved, while by contrast, the mice injected with DMEM became more senile after 4 weeks.

Figs. 12C-E were respectively anatomical picture, H&E staining image of gastric mucosal sections and micro-CT image of the third lumbar vertebra of four mice aged 10 weeks, 25 weeks, 47 weeks (injected with DMEM at the 43^{rd} week and died at the 47^{th} week) and 49 weeks (injected with rFib at the 43^{rd} week and died at the 49^{th} week). It can be observed from these figures that the appearance of the gastrointestinal tract of the mice injected with rFib was similar to that of young mice (aged 10 weeks and 25 weeks); the thickness and density of the gastric mucosa and the structure of the lumbar bone trabecula were similar to those of the 25-week-old mice; by contrast, the aged mice injected with DMEM suffered obvious pathological changes in the gastrointestinal tract and severe fracture at the lumbar bone trabecula, and had short and sparse gastric mucosa.

The analysis results of the obtained micro-CT data were shown in Fig. 12F, from which it can be seen that the aged mice injected with rFib were similar to the 25-week-old mice in bone mineral density (BMD) and relative bone volume (BV/TV), and had improved trabecular number (Tb. N) and trabecular separation/spacing (Tb. Sp) compared to the mice injected with DMEM (5 mice in each group, **p*<0.05, ***p*<0.01, ****p*<0.001, n=5).

The expression levels of *p16 ^{Ink4a}* in different groups of mice were presented in Fig. 12G, where there was no significant difference between the aged mice injected with rFib and the 25-week-old and 10-week-old mice, while the expression level of *p16 ^{Ink4a}* in the aged mice injected with DMEM was increased significantly.

Fig. 12H illustrated the staining results of osteoblasts (ALP) and osteoclasts (TRAP), where compared to the aged mice injected with DMEM, the aged mice injected with rFib exhibited significantly enhanced osteogenic expression and decreased osteoclastic expression.

The quantification results of osteoblasts and osteoclasts were respectively shown in Figs. 12I-J, where the aged mice injected with rFib had similar number of osteoblasts and osteoclasts to the 25-week-old mice.

The lumbar spine of the mice injected with rFib was subjected to immunohistochemical staining, and the results were shown in Fig. 12K. It can be clearly seen that the bones of the mice injected with rFib were positive for human antibodies (hCD29, stained green), and the osteogenic marker Osteocalcin (stained red) was expressed, indicating that the rFib differentiated into osteoblasts in NOD/SCID mice.

Contents of GDF11 (an anti-aging protein) and PDGFA (platelet derived growth factor submit A, promoting the osteogenesis) in the culture medium of rFib were determined and the results were illustrated in Figs. 12L-M, respectively. Specifically, the amount of GDF11 and PDGFA secreted from rFib was significantly higher than that secreted from its homologous Fib, which indicated that the anti-aging and bone density-enhancing functions of rFib may be related to paracrine effects.

Fig. 13 demonstrated that the rFibs were distributed in multiple organs of mice and can differentiate into functional cells.

The distribution of rFib in tissues and organs of mice was detected respectively by fluorescence assay (Fig. 13A) and PCR (Fig. 13B), and the results demonstrated that the rFib was present in the stomach, spleen, lung and liver.

The presence of rFib in lumbar spine was demonstrated by PCR (Fig. 13C), where 1#-5#: mice injected with rFib; 6#-10#: mice injected with DMEM.

The lumbar spine of the mice injected with rFib was subjected to immunohistochemical staining, and the results were shown in Fig. 13D. It can be clearly seen that the bones of the mice injected with rFib were positive for human antibodies (hCD29, stained green), and the osteogenic marker Osteocalcin (stained red) was expressed, indicating that the rFib differentiated into osteoblasts in NOD/SCID mice.

Several other paracrine substances (BFGF, HGF, VEGF) of rFib were determined (Fig. 13E), and the results demonstrated that the rFib was similar to the Fib with respect to the concentration of such substances, which indicated that these paracrine substances were not very associated with the anti-aging mechanism.

### Example 16 Improvement of bone density in aged osteoporotic animals by intravenous injection of rFib

Fig. 14 demonstrated that the rFib can improve the bone density of aged mice with osteoporosis.

Human-derived cells were employed to interfere with the senile osteoporosis in 28-week-old NOD/SCID mice. The experimental group was injected with 1^{∗}10⁶ rFib cells (in 200 µL of DMEM) through the tail vein, and the control group was merely injected with DMEM. The injection was performed once a week and lasted for 3 weeks. The mice were sacrificed 28 days after the first injection, and the lumbar spine was collected for the detection of lumbar bone density. The micro-CT results revealed that the third lumbar vertebrae of the mice in the experimental group were denser than those of the mice in the control group (Fig. 14A).

The micro-CT data indicated that the mice in the experimental group were superior to those in the control group in BMD, BV/TV and Tb. N (Fig. 14B).

### Example 17 Healing effect of culture medium of rFib on animal skin wounds

Full-thickness defect with a width of 8 cm was made on the back of C57 mice. The mice in the control group did not receive treatment, while the mice in the experimental group were smeared with the rFib medium daily.

As shown in Fig. 15, the rFib medium can significantly promote the healing of injured skin, and the wound on the mice treated with rFib medium was almost completely healed 12 days after modeling.

### Example 18 Improvement of rFib for lower extremity ischaemia of mice

The unilateral femoral artery of NOD/SCID mice was ligated to establish the lower extremity ischemia model, and whether the model was successfully established was determined by laser Doppler. 1×10⁶ cells were injected into the ligation point of the femoral artery and its distal and proximal ends, and the blood flow was measured by Laser Doppler respectively 7 and 14 days after injection of cells.

Fig. 16 indicated that the rFib can improve the lower extremity ischemia of mice.

The blood flow in the lower limbs of mice was detected by Laser Doppler, and the results were shown in Fig. 16A, where the rFib and bMSC both can significantly alleviate the lower extremity ischemia.

Gross photographs of the lower limbs of the mice 7 days after the ligation were presented in Fig. 16B, where control: normal lower limb; ischemic: ligated lower limb. The results showed that the ligated lower limbs of the mice were significantly alleviated after treated with rFib or bMSC.

### Example 19 Preparation of rFib using various compound combinations

1. Skin fibroblasts were inoculated to a 6-well plate and cultured in a Fib medium for 24 hours.
2. The Fib medium was replaced with an rFib induction medium containing the small molecular combination Mix Y, and the cells were cultured in the induction medium for 10 days, where the medium was replaced every two days.
3. Then the induction medium was replaced with a HG-DMEM containing 10% FBS or the rFib medium, and the cells were continuously cultured for 3 days and characterized.
5. During the long-term passage, the rFib was cultured in a MSC basal medium and passaged when the confluency reached 90%.

The Fib medium was a HG-DMEM containing 10% FBS or a commercially-available Fibstar-CO medium (cat. no. FMS003C, HCell).

The rFib induction medium was a HG-DMEM supplemented with 10 % FBS, containing 5 µM of Y-27632, 0.2 mM of Vc, 5 µM of EPZ004777, 10 µM of Forskolin, and 1 µM of Repsox, or prepared by introducing 5 µM of Y-27632, 0.2 mM of Vc, 5 µM of EPZ004777, 10 µM of Forskolin, and 1 µM of Repsox to a commercially-available FibGro medium (cat. no. FGS0040, HCell).

The MSC basal medium was a LG-DMEM containing 10% FBS, a commercially-available complete medium for bMSC (cat. no. HUXMA-90011, Cyagen) or an rFib medium (cat. no. CRM0016-01, HCell).

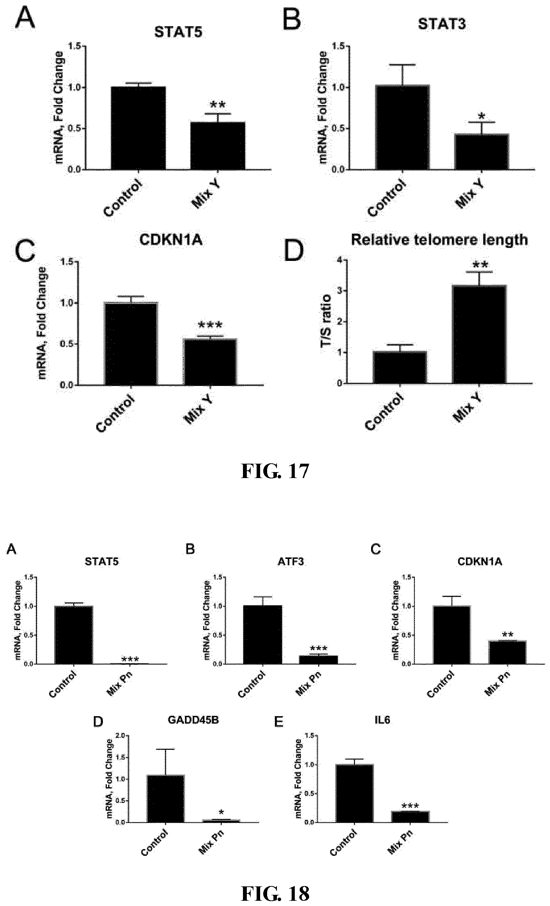

Fig. 17 demonstrated that the treatment with Mix Y can inhibit the expression of STAT5, STAT3 and CDKN1A genes, and extend the telomere, achieving the cell rejuvenation.

### Example 20 Preparation of rFib using various compound combinations

1. Skin fibroblasts were inoculated to a 6-well plate and cultured in a Fib medium for 24 hours.
2. The Fib medium was replaced with an rFib induction medium containing the small molecular combination Mix Pn, and the cells were cultured in the induction medium for 7 days, where the medium was replaced every two days.
3. Then the induction medium was replaced with a HG-DMEM containing 10% FBS or the rFib medium, and the cells were continuously cultured for 3 days and characterized.
5. During the long-term passage, the rFib was cultured in a MSC basal medium and passaged when the confluency reached 90%.

The Fib medium was a HG-DMEM containing 10% FBS or a commercially-available Fibstar-CO medium (cat. no. FMS003C, HCell).

The rFib induction medium was a HG-DMEM supplemented with 10% FBS, containing 0.5 mM of VPA, 3 µM of CHIR99021, 1 µM of Repsox, 10 µM of Forskolin, 5 µM of Go 6983, 5 µM of Y-27632, 0.05 µM of AM580, 5 µM of EPZ004777, 0.2 mM of Vc, 5 µM of TTNPB and 10 µM of 5-Aza-2'-deoxycytidine, or prepared by introducing 0.5 mM of VPA, 3 µM of CHIR99021, 1 µM of Repsox, 10 µM of Forskolin, 5 µM of Go 6983, 5 µM of Y-27632, 0.05 µM of AM580, 5 µM of EPZ004777, 0.2 mM of Vc, 5 µM of TTNPB and 10 µM of 5-Aza-2'-deoxycytidine to a commercially-available FibGro medium (cat. no. FGS0040, HCell).

The MSC basal medium was a LG-DMEM containing 10% FBS, a commercially-available complete medium for bMSC (cat. no. HUXMA-90011, Cyagen) or an rFib medium (cat. no. CRM0016-01, HCell).

As shown in Fig. 18, under the treatment of Mix Pn, the expression of STAT5 was down-regulated, and the expression of ATF3, CDKN1A, GADD45B and IL6 was inhibited, indicating that the cells were rejuvenated.

### Example 21 Preparation of rFib using various compound combinations

1. Skin fibroblasts were inoculated to a 6-well plate and cultured in a Fib medium for 24 hours.
2. The Fib medium was replaced with an rFib induction medium containing a cocktail (Mix Y) of small molecules, and the medium was replaced every two days.
3. After 9 days of the culture, the induction medium was replaced with a HG-DMEM containing 10% FBS, and the cells were continuously cultured for 3-7 days.
4. Then the HG-DMEM containing 10% FBS was replaced with another induction medium containing a small molecular combination Mix Pn2, and the induction medium was replaced every two days.
5. After 7 days of the culture in the induction medium, the induction medium was replaced with a HG-DMEM containing 10% FBS, 10 ng/mL of bFGF, 100 ng/mL of PDGF-AB and 10 ng/mL of BMP4, a HG-DMEM containing 10% FBS or an rFib medium. The cells were cultured for 3 days and then characterized.
6. During the long-term passage, the rFib was cultured in a MSC basal medium and passaged when the confluency reached 90%.

The Fib medium was a HG-DMEM containing 10% FBS or a commercially-available Fibstar-CO medium (cat. no. FMS003C, HCell).

The rFib induction medium containing Mix Y was a HG-DMEM supplemented with 10%FBS, containing 5 µM of Y-27632, 0.2 mM of Vc, 5 µM of EPZ004777, 10 µM of Forskolin and 1 µM of Repsox, or prepared by introducing 5 µM of Y-27632, 0.2 mM of Vc, 5 µM of EPZ004777, 10 µM of Forskolin and 1 µM of Repsox to a commercially-available FibGro medium (cat. no. FGS0040, HCell).

The rFib induction medium containing Mix Pn was a HG-DMEM supplemented with 10% FBS, containing 0.5 mM of VPA, 3 µM of CHIR99021, 1 µM of Repsox, 10 µM of Forskolin, 5 µM of Go 6983, 5 µM of Y-27632, 0.05 µM of AM580, 5 µM of EPZ004777, 0.2 mM of Vc and 5 µM of TTNPB, or prepared by introducing 0.5 mM of VPA, 3 µM of CHIR99021, 1 µM of Repsox, 10 µM of Forskolin, 5 µM of Go 6983, 5 µM of Y-27632, 0.05 µM of AM580, 5 µM of EPZ004777, 0.2 mM of Vc and 5 µM of TTNPB to a commercially-available FibGro medium (cat. no. FGS0040, HCell).

The MSC basal medium was a LG-DMEM containing 10% FBS, a commercially-available complete medium for bMSC (cat. no. HUXMA-90011, Cyagen) or an rFib medium (cat. no. CRM0016-01, HCell).

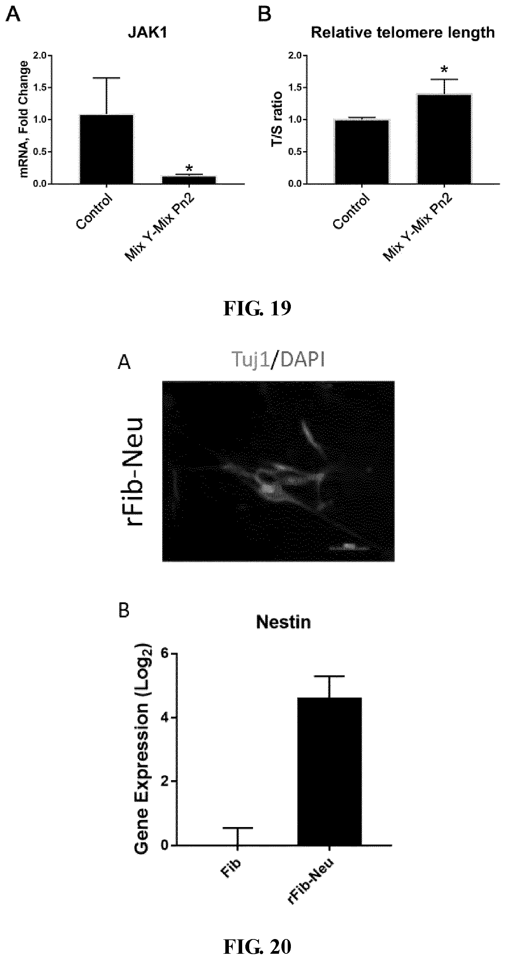

As shown in Fig. 19, the treatment with Mix Y-Mix Pn2 can inhibit the expression of JAK1 and extend the telomere in Fib.

### Example 22 Transdifferentiation of skin fibroblasts into neurons

1. Skin fibroblasts were inoculated to a 6-well plate and cultured in a Fib medium for 24 hours.
2. The Fib medium was replaced with an induction medium containing a small molecular combination Mix Neu, and the medium was replaced every two days.
3. The cells were cultured with the induction medium for 5-12 days, and then it can be observed that the cell morphology was changed from spindle shape to a shape of nerve cells. The induction medium was replaced with a medium for neurons for continuous passage.
4. The transdifferentiated neurons were identified by immunofluorescence assay and quantitative PCR.

The Fib medium was a HG-DMEM containing 10% FBS or a commercially-available Fibstar-CO medium (cat. no. FMS003C, HCell).

The induction medium containing Mix Neu was a HG-DMEM supplemented with 10% FBS, containing 0.5 µM of A8301, 10 ng/mL of bFGF, 5 µM of EPZ004777, 10 µM of RG108, 2 µM of parnate, 10 µM of CHIR99021, 50 µM of Forskolin, 0.5 mM of VPA, 0.05 µM of AM580 and 1 µM of BIX 01294.

The neuron culture medium consisted of 5 mL of DMEM/F12, 5 mL of Neurobasal, 1/100 of N2, 1/50 of B27, 100 µM of cAMP, 20 ng/mL of BDNF, 20 ng/mL of GDNF and 10% KOSR (v/v).

Fig. 20 illustrated the identification results of neurons transdifferentiated from fibroblasts, where Fig. 67: staining of Tuj1 in neurons transdifferentiated from fibroblasts; Fig. 68: measurement of expression level of Nestin.

### Example 23 Differentiation of embryonic stem cells into neurons

1. The adherent embryonic stem cells were digested and then suspended with a neural induction medium.
2. After 10-15 days of culture in the neural induction medium, it can be observed that the cell spheres adhered to the wall. The cell spheres under suspension culture were inoculated to a 6-well plate pretreated with matrigel to perform adherent culture in the neural induction medium for 5-7 days.
3. After the cells adhered to the wall, the neural induction medium was replaced with a neuron culture medium.
4. The induced cells were identified by immunofluorescence staining of neural markers and quantitative PCR.

The neural induction medium was a DMEM/F12 supplemented with 10% KOSR, containing 10 ng/mL of bFGF, 5 µM of Y-27632, 0.5 mM of VPA, 5 µM of EPZ004777, 10 µM of Forskolin and 1 µM of Repsox.

The neuron culture medium consisted of 5 mL of DMEM/F12, 5 mL of Neurobasal, 1/100 of N2, 1/50 of B27, 100 µM of cAMP, 20 ng/mL of BDNF, 20 ng/mL of GDNF and 10% KOSR (v/v).

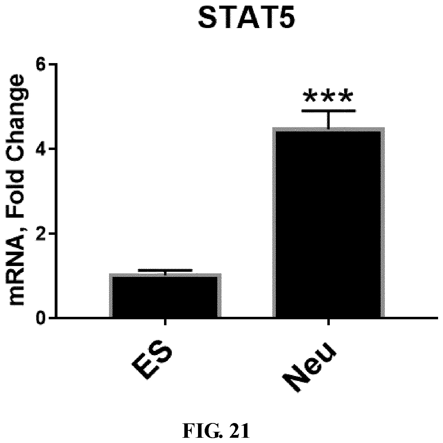

As shown in Fig. 21, under the action of the small molecular combination, the expression of STAT5 gene was up-regulated during the differentiation from ES into neurons.

### Example 24 Characterization of signaling pathways in rFib prepared in Example 1

The rFibs from different donors which were prepared according to the method in Example 1 were subjected to transcriptome sequencing, and a total of 12036 genes in each sample were analyzed by WGCNA to obtain 12 clustering modules.

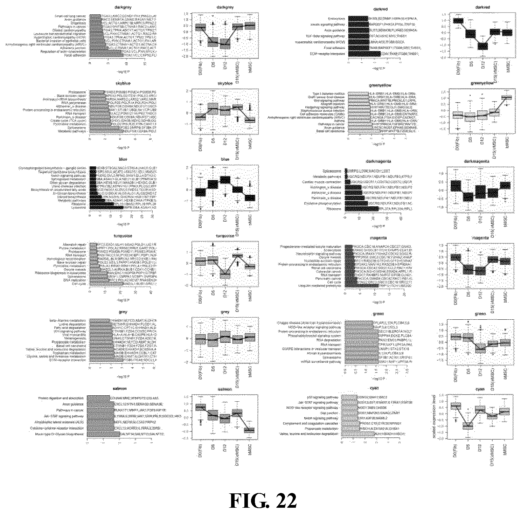

Fig. 22 illustrated the enriched modules in the KEGG pathway, from which it can be seen that a total of 12 modules were enriched in the KEGG pathway, and representative genes of each KEGG pathway were displayed in the order of gene members. The box plot depicted the distribution of average expression level of genes in each module.

## Claims

1. A method of regulating differentiation, dedifferentiation, transdifferentiation, rejuvenation, aging and apoptosis of target cells, comprising:
activating or inhibiting JAK-STAT signaling pathway quantitatively and/or in a timing manner in the target cells.

2. The method according to claim 1, **characterized in that** the activation or inhibition of the JAK-STAT signaling pathway is performed by regulating expression of a gene or protein target;
wherein the gene or protein target is selected from the group consisting of: *CXCL2* (Accession No: AY577905.1), *SOS1* (Accession No: NM_005633.3), *STAT5B* (Accession No: NM_012448.3), *JAK1* (Accession No: NM_001321857.1), *JAK3* (Accession No: NM_000215.3), *SOCS3* (Accession No: NM_003955.4), *IL6ST* (Accession No: NM_001243835.1), *STAT1* (Accession No: NM_007315.3), *STAT2* (Accession No: NM_198332.1), *STAT3* (Accession No: NM_213662.1), *STAT4* (Accession No:NM_001243835.1), *STAT6* (Accession No: NM_001178081.1), *STAT5A* (Accession No: NM_001288720.1), *IRF9* (Accession No: NM_006084.4), *IL6* (Accession No: XM_005249745.5), *IL6R* (Accession No: NM_181359.2), *IL2* (Accession No: NM_000586.3) (such as *IL2A* and *IL2B), PRKCD* (Accession No: NM_001354679.1), *CXCL12* (Accession No: NM_000609.6), *CXCR4* (Accession No: NM_003467.2), *JAK2* (Accession No: NM_004972.3), *IL15RA* (Accession No: NM_001351095.1), *IL20RB* (Accession No: XM_006713665.4), *GHR* (Accession No: NM_001242406.2), *PRLR* (Accession No: NM_001204314.2) and a combination thereof.

3. The method according to claim 1 or 2, **characterized in that** the target cells are selected from the group consisting of fibroblasts, epithelial cells, adipocytes, blood cells, mesenchymal stem cells, nerve cells, muscle cells, cardiomyocytes, smooth muscle cells, vascular endothelial cells, induced pluripotent stem cells, embryonic stem cells, osteoblasts, chondrocytes and osteoclasts of mammals; wherein the target cells are from the subject of mammals, and the mammals are humans, mice, monkeys or pigs; and
cells generated in the regulation of differentiation, dedifferentiation, transdifferentiation, rejuvenation, aging and/or apoptosis of the target cells are obtained.

4. The method according to any one of claims 1-3, **characterized in that** the quantitative activation or inhibition of the JAK-STAT signaling pathway is performed by up-regulating or down-regulating at least one gene or protein involved in the JAK-STAT signaling pathway by 1-300 times or knocking out at least one gene or protein involved in the JAK-STAT signaling pathway.

5. The method according to any one of claims 1-3, **characterized in that** the timing activation or inhibition of the JAK-STAT signaling pathway is performed by regulating expression of at least one gene or protein involved in the JAK-STAT signaling pathway for 24 hours-220 days; and the cells generated from the timing activation or inhibition of the JAK-STAT signaling pathway maintain high, low or no expression of at least one gene or protein involved in the JAK-STAT signaling pathway for a long time, or restore to be the same as the target cells with respect to the expression of the at least one gene or protein involved in the JAK-STAT signaling pathway.

6. The method according to any one of claims 1-5, **characterized in that** the activation or inhibition of the JAK-STAT signaling pathway is performed by regulating at least one of the following pathways or targets: NOD-like receptor signaling pathway, Focal adhesion pathway, cell cycle, tricarboxylic acid cycle (TCA), TGF beta signaling pathway, WNT signaling pathway, Notch signaling pathway, P53 signaling pathway, insulin signaling pathway, calcium signaling pathway, Interleukin-19, Interleukin-20, Interleukin-22, Interleukin-24, IL7, histone deacetylase (HDAC), PKC signaling pathway, RAR pathway, adenylate cyclase signaling pathway, histone methyltransferase (HMT) inhibitors, DNA methyltransferase (DNMT) inhibitors and histone demethylase inhibitors.

7. The method according to claim 6, **characterized in that** the regulation of the NOD-like receptor signaling pathway is performed by regulating expression of a gene or protein target selected from the group consisting of NAIP, IL6, CXCL12, NODI, TAB3, CARD6, CXCL2, CXCL1, CXCL3, CARD8, CARD9, CASP1, CASP12, CASP4, CASP5, NFKB1, TMEM173, TNF, NFKBIB, NOD2, PYDC1, PYCARD, TAB1, TAB2, TNF, TLR4, NLRP1, NLRP12, NLRP3, NLRP6, MCU, RIPK3, RHOA, TAK1, BIRC2, ATG16L1, ATG5, ATG12, TANK and a combination thereof;
the regulation of the Focal adhesion pathway is performed by regulating expression of a gene or protein target selected from the group consisting of TNXB, RAPGEF1, ITGB8, SRC, THBS1, ITGA3, VCL, CAPN2, FLT4, FLT1, ITGA3, ITGB1, ITGB3, ITGB5, ITGB6, ITGB7, ITGA1, ITGA10, ITGA11, ITGA2, ITGA2B, ITGA5, ITGA6, ITGA7, ITGA8, ITGA9, ITGAV, PDRVG, PDGFA, PDGFB, PDGFC, PDGFD, PDGFRA, PDGFRB, BIRC3, BIRC2, BCL2, DOCK1, FN1, HGF, EGF, EGFR, IGF1, IGF1R, VEGFA, VEGFB, VEGFC, CTNNB1 and a combination thereof;
the regulation of the cell cycle is performed by regulating expression of a gene or protein target selected from the group consisting of MAD2L1, BUB1, ORC1, ORC2, ORC3, ORC4, ORC5, ORC6, ATM, ATR, CCNA1, CCNA2, CCNB1, CCNB2, CCNB3, CCND1, CCND2, SMAD2, SMAD3, SMAD4, E2F2, E2F3, E2F4, E2F5, EP300, FZR1, GADD45A, GADD45B, STAG1, STAG2, CDC14A, CDC14B, CDC20, CDC25A, CDC25B, MYC, SMC3, CDC16, YWHAH, YWHAB, YWHAQ, YWHAE, YWHAG, YWHAZ and a combination thereof;
the regulation of the tricarboxylic acid cycle is performed by regulating expression of a gene or protein target selected from the group consisting of IDH3G, IDH3B, MDH2, SDHB, OGDH, MDH1, OGDHL, SUCLG1, SUCLG2, SUCLA2, SDHA, SDHB, SDHC, PDHA1, PDHB, ACLY and a combination thereof;
the regulation of the TGF beta signaling pathway is performed by regulating expression of a gene or protein target selected from the group consisting of ACVR1C, THBS1, FST, TGFB1, TGFBR1, TGFBR2, TGFBR3, BMP4, RUNX3, RUNX2, CREBBP, IFNG, HRAS, FOS, TGFB2, TGFB3, ACVRL1, FOXO3, MTOR, KRAS, CREB1, ATF1, ATF2, ATF4, AKT1, AKT2, AKT3, HNF4A, HNF4G, PIK3R3 and a combination thereof;
the regulation of the WNT signaling pathway is performed by regulating expression of a gene or protein target selected from the group consisting of PRKCA, WNT7B, PRICKLE1, LRP6, CTNNB1, FZD4, CCND2, PRICK, WNT5A, WNT1, WNT10A, WNT11, WNT9A, WNT9B, WNT3, WNT4B and a combination thereof;
the regulation of the Notch signaling pathway is performed by regulating expression of a gene or protein target selected from the group consisting of CIR1, KAT2B, MAML2, PSEN2, DVL2, RFNG, SNW1, DLL4, DTX3, DLL3, DLL1, DTX1, DTX2, CREBBP, CTBP1, CTBP2, JAG1, JAG2, NOTCH1, NOTCH2, NOTCH3, NOTCH4, PSEN1, PSEN2 and a combination thereof;
the regulation of the P53 signaling pathway is performed by regulating a gene or protein target selected from the group consisting of CCNG2, SIAH1, BBC3, TP53AIP1, TP53, SETD7, ATF3, CCNA2, CDK2, CCNG1, CHEK1, PRKCD, KAT2B, PRL23, PPP2CA and a combination thereof;
the regulation of the calcium signaling pathway is performed by regulating a gene or protein target selected from the group consisting of RYR1, RYR2, RYR3, ESR1, AR (androgen receptor), KDR (kinase insert domain receptor), VDR (vitamin D receptor), ITPR1, ITPR2, ITPR3, PDE1A, PDE1B, PDE1C, PRKCA, PRKCD, PRKCE, PRKCG and a combination thereof;
the regulation of the insulin signaling pathway is performed by regulating a gene or protein target selected from the group consisting of RAPGEF1, PHKG1, PYGL, TRIP10, INS, INSR, IRS1, PDPK1, PIK3CA, HRAS, GRB2, PTPN1, PTPN11 and a combination thereof;
the regulation of the PKC signaling pathway is performed by regulating a gene or protein target selected from the group consisting of PRKCA, PRKCB, PRKDC, PRKCZ, PRKCE, PRKCG, PRKCD, PRKCH, PRKCI, PRKCQ, PRKD1, SLC9A5, MAPK3, MAPK9, MAPK8, MAPK1 and a combination thereof;
the regulation of the RAR pathway is performed by regulating a gene or protein target selected from the group consisting of RARA, RARS, RARB, RARG, RXRA, RXRG, FAM120B, NCOA1, NCOR2 and a combination thereof;
the regulation of HDAC is performed by regulating a gene or protein target selected from the group consisting of HDAC1, HDAC2, HDAC3, HDAC4, HDAC5, HDAC6, HDAC7, HDAC8, HDAC9, HDAC10, HDAC11 and a combination thereof;
the regulation of the adenylate cyclase signaling pathway is performed by regulating a gene or protein target selected from the group consisting of PRKAR1A, ADCY10, ADCYAP1, ADCY1, ADCY2, ADCY6, ADCY3, GNAI1, GNAL, GNAT3, PRKACA, PRKAR2B, PRKACB, PRKAR1B, PRKACG, CDKN1B, PRKAR2A, NCAM1, CDKN1A (cyclin dependent kinase inhibitor 1A) and a combination thereof;
the regulation of HMT is performed by regulating a gene or protein target selected from the group consisting of HNMT, DNMT1, KMT2A, EHMT2, EHMT1, KMT2A, DOT1L, EZH2, SETD7, DNMT3B, DNMT3A, SETDB1, SETD2 and a combination thereof;
the regulation of DNMT is performed by regulating expression of a gene or protein target selected from the group consisting of DNMT1, DNMT3B, DNMT3A, CDKN2A, CDKN2B, EHMT2, EHMT1, DNMT3L, CDH1, PARP1, MBD2 and a combination thereof; and
the regulation of histone demethylase is performed by regulating expression of a gene or protein target selected from the group consisting of KDM1A, KDM4A, KDM5A, KDM5B, KDM2A, KDM5C, KDM4B, KDM4C, KDM5D, KDM4D, KDM1B, HISTIH3A, HIST4H4, HIST2H3C, HAT1, HIST1H4C, HIST1H4F, HIST1H4J, HIST1H2AE, HIST1H2BB, CLOCK, NOCA1 and a combination thereof.

8. The method according to any one claims 1-7, **characterized in that** the quantitative activation or inhibition of the JAK-STAT signaling pathway is performed by using a small molecular combination, a cytokine or recombinant protein combination, gene editing, transgenic technique or a combination thereof;
wherein the small molecular combination comprises at least one of a HDAC inhibitor, a TGF-β inhibitor, a PKC inhibitor, a WNT/β-catenin agonist, a cAMP agonist, a RAR agonist, a ROCK inhibitor, a JNK inhibitor, a DNMT inhibitor, a HMT inhibitor, a histone demethylase inhibitor and a JAK-STAT inhibitor;
the HDAC inhibitor is selected from the group consisting of sodium phenylbutyrate, butyrate, sodium butyrate, VPA, Scriptaid, Apicidin, LBH-589(Panobinostat), MS-275, SAHA(Vorinostat), Trichostatin(TSA), Psammaplin A, splitomicin, SRT1720, resveratrol, Sirtinol, APHA, CI-994, Depudecin, FK-228, HC-Toxin, ITF-2357(Givinostat), Chidamide, RGFP 966, PHOB, BG45, Nexturastat A, TMP269, CAY10603, MGCD-0103, Niltubacin, PXD-101 (Belinostat), Pyroxamide, Tubacin, EX-527, BATCP, Cambinol, MOCPAC, PTACH, MC1568, NCH51, TC-H106 and a combination thereof;
the TGF-β inhibitor is selected from the group consisting of 616452, LY2109761, Pirfenidone, Repsox(E-616452), SB431542, A77-01, A8301, GW788388, ITD-1, SD208, SB525334, LY364947, ASP3029, D4476, SB505124 and a combination thereof;
the PKC inhibitor is selected from the group consisting of Go6983, Go6976, Bisindolylmaleimide I (GF109203X) and a combination thereof;
the WNT/β-catenin agonist is selected from the group consisting of MAY-262611, CHIR98014, CHIR99021, LiCl, Li₂CO₃, TD114-2, AZD2858, AZD1080, BIO, Kenpaullone, TWS119, LY2090314, CBM1078, SB216763, AR-A014418 and a combination thereof;
the cAMP agonist is selected from the group consisting of Forskolin, IBMX, Prostaglandin E2 (PGE2), NKH477, 8-pCPT-2'-O-Me-cAMP, GSK256066, Apremilast(CC-10004), Roflumilast, Cilomilast, Rolipram, Milrinone, 8-Bromo-cAMP, Dibutyryl-Camp, Sp-8-Br-cAMPs and a combination thereof;
the RAR agonist is selected from the group consisting of TTNPB, Bexarotene, Ch55, Tamibarotene, Retinol, AM580, ATRA, Vitamin A and its derivatives, 13-cis retinoic acid (RA) and a combination thereof;
the ROCK inhibitor is selected from the group consisting of Y-27632, Y-27632 2HCl, Thiazovivin, Ripasudil(K-115), Fasudil, GSK429286A, RKI-1447, PKI-1313 and a combination thereof;
the JNK inhibitor is selected from the group consisting of SP600125, JNK Inhibitor IX, AS601245, AS602801, JNK-IN-8 and a combination thereof;
the DNMT inhibitor is selected from the group consisting of RG108, Thioguanine, 5-Aza-2'-deoxycytidine (Decitabine), SGI-1027, Zebularine, 5-Azacytidine (AZA) and a combination thereof;
the HMT inhibitor is selected from the group consisting of EPZ004777, EPZ5676, GSK503, BIX 01294, SGC 0946 and a combination thereof;
the histone demethylase inhibitor is selected from the group consisting of parnate(tranylcypromine), Tranylcypromine(2-PCPA)HCl, SP2509, 4SC-202, ORY-1001(RG-6016), GSKJ1, GSK-LSD1 and a combination thereof;
the JAK-STAT inhibitor is selected from the group consisting of STAT5-IN-1, JAK3-IN-1, JAK3-IN-7, WP1066, Homoharringtonine, Pyridone 6, Artesunate, SH-4-54, Baricitinib, Ruxolitinib phosphate, AG-490, Baricitinib phosphate, SAR-20347, CYT387 Mesylate, AS1517499, Peficitinib, Ruxolitinib sulfate, NSC 74859, Stattic, Tofacitinib citrate, Pimozide, Oclacitinib maleate, Ruxolitinib, S-enantiomer, SB1317, Niclosamide, Scutellarin, Solcitinib, Mogrol, Nifuroxazide, TG101348(SAR302503), AG-1478 (Tyrphostin AG-1478) (EGFR inhibitor), KX2-391 (Src inhibitor), PKI-402 (PI3Kα/β/γ/δ and mTOR inhibitor), NSC 74859 (S3I-201) (STAT3 inhibitor), Fludarabine (Fludara) (STAT-1 inhibitor), U0126-EtOH (UO126 EtOH) (MEK1 and MEK2 inhibitor), SGI-1776 free base (Pim1,Pim2 and Pim3 inhibitor), Sorafenib (Nexavar) (VEGFR, PDGFR, c-Raf and B-Raf inhibitor), PLX-4720 (B-RafV600E and c-Raf-1Y340D/Y341D inhibitor) and a combination thereof;
the cytokine or recombinant protein is selected from the group consisting of PDGFAA, PDGFAB, BMP4, IGF1, bFGF, EGF, VEGF, insulin, Activin A, TGF-beta1, Noggin, BMP-2, Shh, IL-6, CXCL10, CXCL12, CXCL2, HGF, IFN gamma, IL-2, IL-6R alpha, IL-2R alpha, TNF-alpha, TNF-beta, TPO, IGF2, IGFBP5, IGFBP6, IGFBP4, IGFBP7, IGFBP9, PDGF-BB, MMP3, GDF11 and TIMP2, and a combination thereof;
the gene editing is performed by using crispr/cas9 and TALEN techniques to up-regulate expression level of target gene or knock out target gene in JAK-STAT signaling pathway, such as STAT5A; and
the transgenic technique is performed by using lentivirus or retrovirus to overexpress or inhibit target gene (e.g.,STAT5A) in the JAK-STAT signaling pathway.

9. The method according to any one of claims 1-8, **characterized in that** a target gene in the Jak-Stat signaling pathway, such as STAT5A, is knocked out to prepare super fibroblasts;
the super fibroblasts are prepared 3-150 days after the STAT5A gene in normal fibroblasts is knocked out, and have rejuvenation characteristics and extended telomere;
the mesenchymal stem cells is treated with a small molecular combination or gene editing to reverse the aging process to obtain rejuvenated mesenchymal stem cells; wherein the small molecular combination comprises at least one of a Jak-Stat inhibitor, a WNT/β-catenin agonist, a DNMT inhibitor, a TGF-β inhibitor and a cAMP agonist; and the gene editing is performed by knocking out target gene in the Jak-Stat signaling pathway, such as STAT5A;
the treatment of the mesenchymal stem cells with the small molecular combination is performed for 1-28 days, wherein the small molecular combination comprises 1-15 µM of CHIR99021 and 1-15 µM of 5-Azacytidine (AZA); 1-15 µM of 5-Azacytidine (AZA) and 3-50 µM of Forskolin; or 1-15 µM of 5-Azacytidine (AZA), 3-50 µM of Forskolin and 1-15 µM of CHIR99021;
cell products generated in the method or derivatives thereof are applied to the manufacture of a kit, a drug, a health-care product, food, cosmetics or a medical device; the preparation of tissue engineering materials as seed cells or scaffold sources, the repairing of injured mammalian tissues and organs and the repairing of aged and degenerated tissues and organs; the medical research or the preparation of an immunomodulator; the *in vitro*/*in vivo* prevention, delaying and reversing of the aging process of mammalian tissues, organs and bodies; or the reprogramming or rejuvenation of cells, tissues, organs and organisms.
